# EUROPEAN PATENT APPLICATION

(11) **EP 4 019 018 A1**
(43) Date of publication of application: **29.06.2022**
(21) Application number: 22157762.0
(22) Date of filing: 09.09.2016
(51) Int. Cl.: A61K 31/4178, A61K 31/216, A61K 31/439, A61K 31/44, A61K 31/4427, A61K 31/4439, A61K 31/517, A61K 31/454, A61K 31/166, A61K 45/06, A61P 25/00, A61P 25/14, A61P 25/16, A61P 25/18, A61P 25/24, A61P 25/28, A61P 43/00, A61K 9/70

(54) **MUSCARINIC COMBINATION AND ITS USE FOR COMBATING HYPOCHOLINERGIC DISORDERS OF THE CENTRAL NERVOUS SYSTEM**

(30) Priority: 11.09.2015 US 201562217081 P; 17.06.2016 US 201662351382 P; 07.07.2016 US 201662359426 P
(62) Divisional of application: 16845090.6
(71) Applicant: Chase Pharmaceuticals Corporation, Washington, District of Columbia, 20006 (US)
(72) Inventor: CHASE, Thomas N., Washington, 20006 (US); CLARENCE-SMITH, Kathleen E., Washington, 20006 (US)
(74) Representative: Boynton, Juliette Alice

(57) **Abstract**

A combination of a muscarinic cholinergic receptor agonist with a non-anticholinergic antiemetic agent, and the optional addition of an acetyl choline esterase inhibitor, for the treatment of hypocholinergic disorder of the central nervous system.

## Description

### CROSS-REFERENCE TO RELATED APPLICATIONS

This application claims priority benefit of United States Provisional Patent Application Serial No. 62/359,426, filed July 7, 2016, United States Provisional Patent Application Serial No. 62/217,081, filed September 11, 2015, and United States Provisional Patent Application Serial No. 62/351,382, filed June 17, 2016; the contents of all of which are incorporated herein by reference in their entirety.

### FIELD OF THE INVENTION

The invention pertains to the field of the treatment of hypocholinergic disorders of the central nervous system, in particular of Alzheimer's Disease (AD), Alzheimer type dementia, Parkinson's dementia, Progressive Supranuclear Palsy (PSP), Mild Cognitive Impairment (MCI), Lewy body disease, Frontotemporal lobe dementia (FTD), Frontotemporal lobar degeneration, Pick's disease, Post-stroke dementia, Vascular dementia, Traumatic brain injury (TBI), Senile dementia, Autism, anorexia nervosa, falls, post-operative delirium, Down Syndrome, chronic neuropathic pain, schizophrenia, Tourette syndrome, Tardive dyskinesia, Huntington's disease, Friedrich's ataxia, Cognitive Impairment associated with Multiple Sclerosis, and other disorders of the nervous system involving a deficit in acetyl-choline neurotransmission; and to a new competitive and safe treatment, a combination comprising a cholinergic agonist agent and a non-anticholinergic antiemetic agent. More particularly, the invention proposes a combination of a muscarinic agonist which is a Muscarinic Cholinergic Receptor Agonist (MCRA) and an antiemetic agent, as well as the optional addition of an acetyl choline esterase inhibitor.

### DEFINTIONS

- "CNS": Central Nervous System.
- "PNS": Peripheral Nervous System.
- "Muscarinic type receptors (mAChRs)": Five subtypes of muscarinic receptors, M1 through M5, have been identified.
- "MCRA(s)": Cholinergic Receptor Agonist(s) acting on the mAChRs, including orthosteric activators and allosteric activators, in particular both allosteric agonists and positive allosteric modulators, of mAChR subtypes.
- "naAEA(s)": non-anticholinergic Anti-Emetic Agent(s).
- "Non-anticholinergic" refers to antiemetic medications not primarily regarded as anticholinergic agents; they are entirely devoid of anticholinergic activity or have an extremely low ability to prevent acetylcholine from acting at its cholinergic receptor sites.
- "MTD": maximum (or maximal) tolerated dose, i.e. the highest dose of a drug or treatment that does not cause unacceptable side effects. The maximum tolerated dose is determined in clinical trials by testing increasing doses on different groups of people until the highest dose with acceptable side effects is found (NCI Drug Dictionary).
- "CSF": Cerebrospinal Fluid.
- "IR": Immediate Release of the active ingredient from a composition.
- "ER": Extended Release, including sustained release, controlled release and slow release of the active ingredient from a composition by any administration route, in particular, but not limited to oral and parenteral (including transcutaneous, transdermal, intramuscular, intravenous, and subcutaneous routes).
- "AChE": Acetyl Choline esterase
- "AChEI(s)": Acetyl Choline Esterase Inhibitor(s).
- "Transdermal delivery": administration of drug via the skin which targets, without limitation, skin tissues just under the skin, other tissues or organs under the skin, systemic circulation, and/or the central nervous system.
- "Transdermal Therapeutic System (TTS)": administration of drug via transdermal delivery using transdermal drug formulations and transdermal patches incorporating such transdermal drug formulations.
- "comprising" means that the compositions and methods include the recited elements, but do not exclude others. "comprising" is inclusive of the terms "consisting of" and "consisting essentially of".
- "consisting essentially of" means that the methods and compositions may include additional steps, components or ingredients, but only if the additional steps, components or ingredients do not materially alter the basic and novel characteristics of the claimed methods and compositions. In certain embodiments, "consisting essentially of" means that the subsequently named component(s) is necessarily included but that another unlisted ingredient(s) that does not materially affect the basic and novel properties can also be present. For example, when used to define compositions and methods, "consisting essentially of" means excluding other elements of any essential significance to the combination for the intended use. Thus, for example, a composition consisting essentially of the elements as defined herein would not exclude trace contaminants and pharmaceutically acceptable carriers.
- "pharmaceutically acceptable salt" means either a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.
- "combination therapy" means treating a patient with the combination of a cholinergic agonist agent and a non-anticholinergic antiemetic agent. Combination therapy may include a temporal overlap of other therapeutic agents, depending on the clinical course of a given hypocholinergic disease in a subject.

### BACKGROUND OF THE INVENTION

Reduced levels of neurotransmitters including acetylcholine occur in dementias of the Alzheimer type. In particular, a deficit in acetylcholine-mediated transmission is thought to contribute to the cognitive and neurobehavioral abnormalities associated with these disorders. Accordingly, drugs known to augment cholinergic transmission in the CNS are the mainstay of current therapy. In addition, other diseases of the nervous system also involve decreased cholinergic transmission and are referred to as "hypocholinergic syndromes and disorders of the Central Nervous System (CNS)". In addition to AD, and AD-type dementia, such diseases, herein below referred to as "hypocholinergic disorders" also include, but are not limited to, Alzheimer's Disease (AD), AD-type dementia, Parkinson's dementia, Progressive Supranuclear Palsy (PSP), Mild Cognitive Impairment (MCI), Lewy Body Disease (LBD), Parkinson disease dementia (PDD), Frontotemporal lobe dementia (FTD), Frontotemporal lobar degeneration, Pick's disease, post-stroke dementia, vascular dementia, Traumatic Brain Injury, Senile dementia, Autism, Anorexia Nervosa, falls, post-operative delirium, Down syndrome, Tourette syndrome, tardive dyskinesia, Pick's disease, Huntington's disease, Friedrich's ataxia, chronic neuropathic pain, schizophrenia, Cognitive Impairment associated with Multiple Sclerosis, and other disorders of the nervous system involving a deficit in acetyl-choline neurotransmission. It is well documented that schizophrenic patients experience cognitive disturbances that are not well addressed by current medications (reviewed in Foster et al, Neuropsychiatric Disease and Treatment, 2014:10 183-191).

MCRAs have been reported to dose-dependently improve the cognitive disturbances associated with schizophrenia, but the effect of MCRAs is of limited magnitude and dose-dependent side effects prevent further increases in the MCRA doses.

Acetylcholinesterase inhibitors (AChEIs) are now not only part of the standard of care for patients suffering from a dementia of the Alzheimer type, but are also widely used off-label for various other chronic often progressive hypocholinergic disorders of the nervous system. As a general mechanism of action, AChEIs enhance acetylcholine-mediated neurotransmission. All act in the human CNS to increase and prolong the availability of acetylcholine by inhibiting its degradatory enzyme acetylcholinesterase (AChE). Four AChEIs have been approved by the U.S. FDA for the treatment of dementias of the Alzheimer type: tacrine, donepezil [Aricept^{®}], rivastigmine [Exelon^{®}] and galantamine [Razadyne^{®}]. Rivastigmine has also been approved for the treatment of Parkinson's disease dementia. AChEIs are available in various formulations including immediate release forms such as tablets, capsules and solutions as well as rapid dissolving and extended release forms for oral administration as well as those for parenteral (e.g. transdermal) administration.

Unfortunately, however, none of the available AChEIs offers more than modest clinical benefit for patients suffering from any of the aforementioned dementing disorders, as traditionally administered, even when these medications are administered at their maximum safe and tolerated doses.

Use of AChEIs with a non-anticholinergic antiemetic agent has been described (US 8,877,768, the disclosure of which is incorporated herein by reference in its entirety).

Another way to increase the cholinergic transmission in the brain is to stimulate post-synaptic cholinergic receptors by administering an agonist of the muscarinic receptors, but the results were generally disappointing.

In fact, many MCRAs have been studied in the last two decades but, except for cevimeline (EVOXAC^{®}), which is marketed in the U.S.A. for the limited indication of the treatment of symptoms of dry mouth in patients with Sjogren's Syndrome, none of the MCRAs showed a significant activity on the CNS which could be used for the treatment of Alzheimer type dementia or of hypocholinergic disorders.

In a primate study, the muscarinic ligand (5R,6R)-6-(3-butylthio-1,2,5-thiadiazol-4-yl)-1-azabicyclo[3.2.1]octane (BuTAC) exhibited high affinity for muscarinic receptors, but induced vomiting that was mitigated by administration of domperidone (M.B. Andersen et al. Neuropsychopharmacology 2003; 28:1168-1175). No trial in humans with this muscarinic agonist apparently followed this study.

The (*E*)-N-methoxy-1-(1-methyl-1,2,5,6-tetrahydropyridin-3-yl)methanimine, a non-selective muscarinic acetylcholine receptor partial agonist with cognitionacting properties known as milameline and described in US 6,037,347, the disclosure of which is incorporated herein by reference in its entirety, was investigated for the treatment of Alzheimer's disease. However, the drug, while possessing a pharmacological profile consistent with that of a muscarinic partial agonist, produced central cholinergic action in rats and monkeys at doses slightly higher than those stimulating peripheral cholinergic receptors (Schwarz RD, Callahan MJ, Coughenour LL, Dickerson MR, Kinsora JJ, Lipinski WJ, Raby CA, Spencer CJ, Tecle: "Milameline (CI-979/RU35926): a muscarinic receptor agonist with cognitionactivating properties: biochemical and in vivo characterization"; J Pharmacol Exp Ther. 1999 Nov;291(2):812-22 - Schwarz 1999, the disclosure of which is incorporated herein by reference in its entirety). The development of milameline seems to have been discontinued.

Similarly, the (3R)-N-methoxyquinuclidine-3-carboximidoyl cyanide hydrochloride known as sabcomeline and described in US 5,278,170, the disclosure of which is incorporated herein by reference in its entirety, is a selective M1 receptor partial agonist that was under development for the treatment of Alzheimer's disease (Loudon JM, Bromidge SM, Brown F, et al.: "SB 202026: a novel muscarinic partial agonist with functional selectivity for M1 receptors"; J Pharmacol. Exp. Ther. 1997 Dec;283(3): 1059-68 - Louden 1997, the disclosure of which is incorporated herein by reference in its entirety). It was submitted to phase III clinical trials before being discontinued (R & D Focus Drug News, March 8, 2004).

Another MCRA, the 5-[4-(hexylsulfanyl)-1,2,5-thiadiazol-3-yl]-1-methyl-1,2,3,6-tetrahydropyridine, known as tazomeline and described in US 5,041,455, the disclosure of which is incorporated herein by reference in its entirety, acts as a non-selective muscarinic acetylcholine receptor agonist. It was in clinical trials for the treatment of cognitive dysfunction such as that seen in Alzheimer's disease and schizophrenia, but, according to Wikipedia (September 9, 2015), its "development was apparently scrapped for unknown reasons" and no sign of an effective development is known.

A close analog of tazomeline, the 3-(4-hexyloxy-1,2,5-thiadiazol-3-yl)-1-methyl-5,6-dihydro-2H-pyridine known as xanomeline and described in US 5,043,345, the disclosure of which is incorporated herein by reference in its entirety, has been disclosed as a muscarinic acetylcholine receptor agonist with reasonable selectivity for the M1 and M4 subtypes. The efficacy of xanomeline, which stimulates muscarinic receptors in the brain and in the periphery was studied in patients with Alzheimer disease in a 6-month double-blind, placebo-controlled, parallel group trial. Compared to placebo, xanomeline was shown to significantly improve cognitive and behavioral symptoms of Alzheimer disease (Bodick et al, Arch Neurol 1997; 54(4):465-73; Shekhar et al, Am. J. Psychiatry, 2008, 165(8):1033-1039), but also caused dose-dependent unacceptable side effects, including bradycardia, gastro-intestinal distress, excessive salivation, and sweating. Such side effects prevented the use of doses of xanomeline that could achieve maximum anti-dementia efficacy and reflect stimulation of cholinergic receptors outside the brain.

Xanomeline is also described in a transdermally administrable form in US 5,980,933, the disclosure of which is incorporated herein by reference in its entirety, and a clinical experimentation of said preparation was announced. The paper Mirza, N. et al., CNS Drug Review, 9(2): 159-186 (2003) confirmed a phase II clinical trial with transdermal xanomeline, but no specific result appeared in the literature after that date.

A xanomeline fluorinated analog, the 3-[3-(3-(3-fluorophenyl)-2-propyn-1-ylthio)-1,2,5-thiadiazol-4-yl]-1,2,5,6-tetrahydro-1-methylpyridine oxalate, known as EUK 1001, was disclosed by Xiaoping Lei in CN 1821243B and considered a promising therapeutic agent for the treatment of AD and age-related memory disorders (Yihui Cui, Dong Wang, Wen Si, Wen Lv, Yan Niu, Xiaoping Lei, Yinhe Hu and Xiaohua Cao: "Enhancement of memory function in aged mice by a novel derivative of xanomeline"; Cell Research; 2008; 18:1151-1153 published online 21 October 2008 - Yihui Cui 2008 the disclosure of which is incorporated herein by reference in its entirety). However, no results of clinical trials in human beings using EUK1001 have been reported in the literature.

Dose-limiting adverse events attending the use of drugs that stimulate cholinergic transmission, such as xanomeline, appear to primarily reflect the excessive stimulation of peripheral cholinergic receptors, especially those of the muscarinic type (mAChRs), such that in both healthy volunteers and Alzheimer's patients many of these side effects have been reported for xanomeline; in the patient population this led to a discontinuation rate higher than 50% while the effects on cognition were not robust and mainly seen at the highest doses tested (Mirza2003).

As a matter of fact, for the MCRAs tested in clinical trials for the treatment of Alzheimer disease,
- the milameline maximum tolerated dose was determined as being 2 mg four times per day (J.J. Sramek et al. Life Sciences 1998, 62/3: 195-202,);
- the xanomeline minimum intolerated dose was reached at 115 mg three times a day, and 100 mg three times a day was defined as the MTD by J.J. Sramek et al. (J Clin Pharmacol 1995;35:800-806), who also observed that higher xanomeline concentrations appear to be associated with reduced tolerance to the drug; the literature also shows that, in a 6-month double-blind, placebo-controlled, parallel group efficacy study, 59% of patients discontinued treatment after receiving 75 mg xanomeline orally three times daily, mainly because of adverse events, predominantly gastrointestinal (N. R. Mirza et al. CNS Drug Reviews Vol. 9, No. 2, pp. 159-186, 2003).

In conclusion, the development of all of the above MCRAs was discontinued because the results of the studies were disappointing not due to a basic muscarinic inactivity of the products but because said products had limited efficacy at doses that were tolerable in patients, and induced dose-limiting, intolerable adverse effects at higher doses.

In a review published in NEUROLOGY, 49, July 1997, by H. Robert Brashear, MD, of the book "Muscarinic Agonists and the Treatment of Alzheimer Disease" (Edited by Abraham Fisher - R.G. Landes ,1996), the reviewer concluded his comments as follows: "It will be of interest to most clinicians who treat Alzheimer's disease and valuable to chemical researchers, basic neuroscientists, biochemists, and pharmacologists investigating cholinergic dysfunction and therapy*".* Despite this clear interest and the extensive studies made on a series of compounds during the last two decades, none of the studied compounds became a drug for the treatment of this disease for the reasons set forth above.

In addition, MCRAs consisting of allosteric modulators of the M₁-muscarinic acetylcholine receptor have been extensively studied and are the object of copious patents and scientific literature.

A review by B.J. Melancon, J.C Tarr, J.D. Panarese, M.R. Wood and C.W. Lindsley published in Drug Discovery Today; Volume 18, Numbers 23/24, December 2013, "Allosteric modulation of the M1 muscarinic receptor: improving cognition and a potential treatment for schizophrenia and Alzheimer's disease" (Melancon et al.), the disclosure of which is incorporated herein by reference in its entirety, illustrates the role of the M₁ receptor in Alzheimer's disease and in schizophrenia by referring to selected allosteric modulators of the M₁ receptor.

This review also reports that the positive allosteric modulator MK-7622 entered Phase II clinical trials as an adjunct therapy to AChEIs in patients with AD This positive allosteric modulator of the M₁ receptor, 3-[(1S,2S)-2-hydroxycyclohexyl]-6-[(6-methylpyridin-3-yl)methyl]benzo[h]quinazolin-4(3H)-one, is described in US 8,883,810, the disclosure of which is incorporated herein by reference in its entirety.

The precise causes of the vomiting and related gastrointestinal symptoms induced by cholinergic therapy are not known.

An improvement in the treatment of Alzheimer type dementia is attained by a combined therapy associating a non-selective, peripheral anticholinergic agent, at a dose of from 20% to 200% the current daily doses, with an AChEI, at a dose up to about 6 times the maximal recommended dose of said AChEI, as disclosed in US 8,404,701, the disclosure of which is herein incorporated by reference in its entirety. By such a treatment, a higher acetylcholinesterase inhibition in the CNS is achieved and greater relief of the symptoms of Alzheimer type dementia is enabled, by concomitantly decreasing concurrent adverse effects. This result was obtained by successfully inferring that the good dose-response obtained with the AChEIs, i.e. with enzyme inhibitors, would allow an increase of the inhibition of AChE in the CNS with a safe increase of the AChEI dose. Conversely, in the case of the muscarinic receptors, nothing in the literature suggests how to effectively take advantage of the properties of MCRAs. In particular, the literature does not give any indication or suggestion for exploiting the potentiality of said muscarinic agonists in the treatment of disease.

US 8,877,768, the disclosure of which is herein incorporated by reference in its entirety, discloses a combined therapy associating a non-anticholinergic-antiemetic agent, at a dose of from 50% to 300% the current IR daily doses, with an AChEI, at a dose up to 4 times the maximal recommended doses of said AChEI when administered alone.

US 8,883,810 (see also WO 2010/059773), the contents of which are incorporated herein in their entirety for reference, describing MK-7622, cites the combination of a class of aryl methyl benzoquinazolinone compounds disclosed therein with other drugs to render the administration safer or more effective or to reduce the risk of side effects or toxicity of said aryl methyl benzoquinazolinones. These combinations include anticholinergic drugs but the document does not disclose any non-selective, peripheral anticholinergic drug. On the contrary, it specifically cites biperiden and trihexyphenidyl hydrochloride as anticholinergics, both being central anticholinergic agents for the treatment of the Parkinson's disease. Centrally acting anticholinergics would block the beneficial effects of MCRAs on hypocholinergic disorders of the brain.

However, the problem of dose-limiting adverse effects encountered during the clinical trials involving MCRAs, which can be expected to occur with any muscarinic receptor agonist remains unsolved.

### SUMMARY OF THE INVENTION

Humans are exquisitely sensitive to the peripheral side effects of drugs that enhance cholinergic transmission. The present invention is based on the inventors' observation that all the MCRAs studied cause vomiting in humans in doses lower than the doses that will significantly increase cholinergic transmission in the brain. Animals are different from humans in this respect (at least the animals that have been tested). The present invention provides a solution to this problem, by providing a combination of MCRAs with drugs that block vomiting without blocking the effects of said MCRAs in the brain. More particularly, according to the present invention it is possible to safely treat a human being, in particular a patient suffering from a hypocholinergic disorder, with the combination of a naAEA and a MCRA, thus enabling the therapeutic potential of said MCRA According to the invention the therapeutic potential of said MCRA is especially realized when the MCRA is administered at a dose that is even higher than, preferably up to 3-6 times higher than the MCRA dose causing vomiting and also even higher than, preferably up to 3-6 times higher than the Maximum Tolerated Dose determined in the clinical trials of said MCRA used alone. Said result is achieved by administering a naAEA in combination with single MCRA doses that may be from 1-time to 6 times, advantageously from 1.2-times to 6-times, normally from 1.2 times to 4 times higher than the maximum amount contained in the commercial products at the time of filing or of the maximal, single MCRA dose administered during the clinical trials,

The present inventors have found that the aforementioned observation of J.J. Sramek et al. (J Clin Pharmacol 1995;35:800-806) concerning the apparently inseparable muscarinic activity/adverse effects relationship may be annulled, while on one side preserving the full therapeutic muscarinic activity of the agonist in the brain and on the other side by eliminating the dose-limiting adverse effects, by combining said muscarinic agonist with a naAEA.

In fact, it has been found that said naAEA surprisingly acts to attenuate the dose-limiting side effects of MCRAs, thus enabling a safe, greater increase in the MTD of MCRAs and consequent increase in the efficacy of MCRAs (said increase being contrary to what was observed by Sramek et al.(J Clin Pharmacol 1995;35:800-806)), thus permitting the utilization of the full, real potency of the muscarinic agonist.

In particular, it has been found that an antiemetic allows the safe administration of MCRAs at doses - for the MCRAs already submitted to clinical investigation - never attained heretofore. In particular, said antiemetic, when concurrently or sequentially administered in combination with a MCRA, is able not only to neutralize the adverse effects that hindered the development of a muscarinic agonist for the treatment of central disorders due to a deficit of acetylcholine in the brain, but also to increase the concentrations of acetylcholine in the CNS.

Thus, by using an antiemetic, safe administration of even high doses of a MCRA can be achieved for a patient suffering from hypocholinergic disorders of the central nervous system, such as Alzheimer's disease (AD), AD-type dementia, Progressive Supranuclear Palsy (PSP), Mild Cognitive Impairment (MCI), Lewy Body Disease (LBD), Frontotemporal lobe dementia (FTD), Frontotemporal lobar degeneration, Parkinson disease dementia (PDD), post-stroke dementia, vascular dementia, Traumatic Brain Injury (TBI), Senile dementia, Autism, Anorexia Nervosa, Down syndrome, chronic neuropathic pain, Tourette syndrome, tardive dyskinesia, Pick's disease, Huntington's disease, Friedrich's ataxia, falls, post-operative delirium, schizoaffective disorders, schizophrenia, Cognitive Impairment associated with Multiple Sclerosis, and other disorders of the nervous system involving a deficit in acetyl-choline neurotransmission. The combination allows said MCRA to safely activate the acetylcholine receptors and to improve cognition.

The finding of the present invention represents further progress in the treatment of hypocholinergic disorders, especially in view of the lack of efficacy of the muscarinic cholinergic receptor agonists at the doses administered to the patients and of the apparently irreducible adverse effects induced by said agonists at the administered doses.

This finding of the present invention eliminates the dose-limit that, in the past, caused the failure of all the clinical trials, thus providing a method for treating Alzheimer type dementia as well as hypocholinergic disorders of the CNS by enabling the full efficacy of MCRAs to be achieved. The method of the present invention comprises treating a patient in need of such a treatment with an antiemetic, in combination with a MCRA. This treatment method precludes the onset of MCRA-associated peripheral dose-limiting adverse effects.

Thus, the present invention provides a combination comprising, as Components:
(a) a muscarinic cholinergic receptor agonist (MCRA); and
(b) a non-anticholinergic antiemetic agent (naAEA).

The present invention also provides a method of using the above combination for the treatment of hypocholinergic disorders in CNS.

In addition, the present invention provides the above combination wherein said Components (a) and (b) are formulated in the same unit form, in a fixed-dose combination.

Furthermore, the present inventors found that, in order to assure safe treatment of hypocholinergic disorders, the non-anticholinergic antiemetic agent should be administered in combination with the MCRA from the beginning of the therapeutic treatment of a patient submitted to this cholinergic treatment for the first time and that said combination is essential for the continuation of said therapeutic treatment. In order to assure said safe treatment, said naAEA and said MCRA may advantageously be in a fixed-dose combination consisting of a pharmaceutical composition wherein said naAEA and said MCRA are formulated in a dosage unit form in admixture with a pharmaceutical carrier.

Thus, the present invention also provides the above combination, wherein said Components (a) and (b) are formulated in the same unit form. Herein below, the (a)+(b) fixed-dose combination will also designated as "Component (a/b)".

According to the present invention, the novel pharmaceutical composition in dosage unit form comprising, as active ingredients, (a) a MCRA; and (b) a naAEA, in admixture with a pharmaceutical carrier or vehicle, is a particularly advantageous embodiment of the present invention.

A further particularly advantageous embodiment is a pharmaceutical composition in dosage unit form comprising as active ingredients:
(a) a MCRA selected from the group consisting of xanomeline and pharmaceutically acceptable salts thereof and MK-7622 and pharmaceutically acceptable salts thereof, at a dose capable of increasing the acetylcholine supply in the CNS of a patient suffering from a hypocholinergic disorder; and
(b) a naAEA selected from the group consisting of ondansetron and pharmaceutically acceptable salts and solvates thereof, domperidone and pharmaceutically acceptable salts and solvates thereof, metoclopramide and pharmaceutically acceptable salts and solvates thereof, in an amount of from 50% to 300% the maximum amount contained in the commercial preparations of said naAEA at the time of this filing for the treatment of nausea/vomiting, in admixture with a pharmaceutical carrier.

The present invention also provides the addition of an AChEI to the above MCRA/naAEA combination, thus assuring a maximum supply of acetylcholine to the CNS by the administration of a combination of the three components. Herein below, the third AChEI component will also be designated as "Component (c)".

### DETAILED DESCRIPTION

The present invention provides a pharmaceutical combination comprising as Components:
(a) a muscarinic receptor agonist selected from the group consisting of muscarinic cholinergic receptor agonists (MCRA); and
(b) a naAEA; and optionally
(c) an AChEI.

According to an embodiment, said pharmaceutical combination comprises as Components:
(a) a MCRA, in a pharmaceutical composition in admixture with a pharmaceutical carrier; and
(b) a naAEA, in a pharmaceutical composition in admixture with a pharmaceutical carrier.

According to another embodiment, said pharmaceutical combination comprises as Components:
(a) a MCRA, in a pharmaceutical composition in admixture with a pharmaceutical carrier; and
(b) a naAEA selected from the group consisting of ondansetron and pharmaceutically acceptable salts and solvates thereof, in a pharmaceutical composition in admixture with a pharmaceutical carrier.

According to this embodiment, an advantageous pharmaceutical combination comprises, as Components:
(a) a MCRA selected from the group consisting of cevimeline and pharmaceutically acceptable salts thereof, milameline and pharmaceutically acceptable salts thereof; xanomeline and pharmaceutically acceptable salts thereof, and MK-7622 and pharmaceutically acceptable salts thereof, in a pharmaceutical composition in admixture with a pharmaceutical carrier; and
(b) a naAEA which is ondansetron hydrochloride dihydrate, in a pharmaceutical composition in admixture with a pharmaceutical carrier.

A specific pharmaceutical combination according to this embodiment comprises, as Components
(a) a MCRA selected from the group consisting of cevimeline and pharmaceutically acceptable salts thereof, in an amount (in cevimeline) of from 34.5 mg to 180 mg; milameline and pharmaceutically acceptable salts thereof, in an amount (in milameline) of from 2.4 mg to 12 mg; xanomeline and pharmaceutically acceptable salts thereof, in an amount (in xanomeline) of from 90 mg to 450 mg; and MK-7622 and pharmaceutically acceptable salts thereof, in an amount (in MK-7622) of from 5 mg to 270 mg, in a pharmaceutical composition in admixture with a pharmaceutical carrier; and
(b) an naAEA which is ondansetron hydrochloride dihydrate in an amount (in ondansetron) of from 4 mg to 64 mg, in a pharmaceutical composition in admixture with a pharmaceutical carrier. Preferably, in this specific combination the MCRA is cevimeline hydrochloride hemihydrate.

According to a further embodiment, said pharmaceutical combination comprises as Components:
(a/b) a pharmaceutical composition in dosage unit form comprising
   (a) a MCRA; and
   (b) a naAEA,
   in admixture with a pharmaceutical carrier; and
(c) an AChEI, in admixture with a pharmaceutical carrier.

A preferred pharmaceutical combination comprises
(a) a MCRA selected form the group consisting of cevimeline and pharmaceutically acceptable salts thereof; milameline and pharmaceutically acceptable salts thereof; xanomeline and pharmaceutically acceptable salts thereof and MK-7622 and pharmaceutically acceptable salts thereof, in a pharmaceutical composition in admixture with a pharmaceutical carrier;
(b) a naAEA selected from the group consisting of ondansetron and pharmaceutically acceptable salts and solvates thereof, in a pharmaceutical composition in admixture with a pharmaceutical carrier; and
(c) an AChEI consisting of rivastigmine in admixture with a pharmaceutical carrier in a patch for transdermal administration.

This combination may be used for the treatment of Alzheimer type dementia and more generally for hypocholinergic disorders of the central nervous system, including, but not limited to, Alzheimer's Disease (AD), Alzheimer type dementia, Parkinson's dementia, Progressive Supranuclear Palsy (PSP), Mild Cognitive Impairment (MCI), Lewy body disease, Frontotemporal lobe dementia (FTD), Frontotemporal lobar degeneration, Pick's disease, Post-stroke dementia, Vascular dementia, Traumatic brain injury (TBI), Senile dementia, Autism, anorexia nervosa, falls, post-operative delirium, Down Syndrome, chronic neuropathic pain, schizophrenia, Tourette syndrome, Tardive dyskinesia, Huntington's disease, Friedrich's ataxia, Cognitive Impairment associated with Multiple Sclerosis, and other disorders of the nervous system involving a deficit in acetyl-choline neurotransmission.

### The MCRAs Component (a)

Any MCRA which is able to cross the brain blood barrier of a human in order to stimulate the muscarinic cholinergic receptors in the CNS may be used as Component (a) according to the present invention.

Advantageously, the MCRA used as Component (a) is one of the muscarinic cholinergic agonists that have extensively, but unsuccessfully been investigated in relation to the possibility of using them for the treatment of Alzheimer type dementia, as well as M₁ receptor positive allosteric modulators that are believed to be useful in the treatment of this and other diseases involving the muscarinic M₁ receptor.

Preferably, said MCRA is selected from the group consisting of
- 1-methylpiperidine-4-spiro-5'(2'-ethyl-1',4'-thiazoline-3'-one) (AF267) and pharmaceutically acceptable salts and solvates thereof, especially its hydrochloride (AF 267B) described in EP0711292;
- cis-2'-methylspiro {1-azabicyclo [2.2.2] octane-3,5'-[1,3] oxathiolane} (cevimeline) described in US4,855,290 and US5,571,918, and pharmaceutically acceptable salts and solvates thereof, especially its hydrochloride hemihydrate;
- 3-[3-(3-(3-fluorophenyl)-2-propyn-1-ylthio)-1,2,5-thiadiazol-4-yl]-1,2,5,6-tetrahydro-1-methylpyridine described in CN 1821243B and pharmaceutically acceptable salts and solvates thereof, especially its oxalate (EUK 1001);
- (*E*)-N-methoxy-1-(1-methyl-1,2,5,6-tetrahydropyridin-3-yl)methanimine (milameline) described in US 6,037,347 and pharmaceutically acceptable salts and solvates thereof, especially its hydrochloride;
- 2-ethyl-8-methyl-2,8-diazaspiro[4.5]decane-1,3-dione described in US 3,056,796 (RS-86) and pharmaceutically acceptable salts and solvates thereof, especially its hydrobromide;
- (3*R*)-N-methoxyquinuclidine-3-carboximidoyl cyanide (sabcomeline) described in US 5,278,170 and pharmaceutically acceptable salts and solvates thereof, especially its hydrochloride;
- (3*R*)-3-(prop-2-yn-1-yloxy)-1-azabicyclo[2.2.2]octane (talsaclidine) described in US 5,286,864 and pharmaceutically acceptable salts and solvates thereof, especially its fumarate;
- 5-[4-(hexylthio)-1,2,5-thiadiazol-3-yl]-1-methyl-1,2,3,6-tetrahydropyridine (tazomeline) described in US 5,041,455 and pharmaceutically acceptable salts and solvates thereof, especially its hydrochloride;
- 3-(4-hexyloxy-1,2,5-thiadiazol-3-yl)-1-methyl-5,6-dihydro-2H-pyridine (xanomeline) described in US 5,043,345 and EP 0384288 and pharmaceutically acceptable salts and solvates thereof, especially its oxalate and L-hydrogen tartrate;
- (4-n-butyl-1-[4-(2-methylphenyl)-4-oxo-1-butyl]-piperidine (AC-42) and pharmaceutically acceptable salts and solvates thereof, especially its hydrogen chloride;
- (5R,6R)-6-(3-butylthio-1,2,5-thiadiazol-4-yl)-1-azabicyclo[3.2.1]octane (BuTAC), mentioned above, and pharmaceutically acceptable salts thereof, described for example as squalene synthetase inhibitor and anti-hypercholesterolemic agent in US 5,750,538;
- 1-[1'-(2-methylbenzyl)-1,4'-bipiperidin-4-yl]-1,3-dihydro-2H-benzimidazol-2-one (TBPB) and pharmaceutically acceptable salts and solvates thereof;
- 4-Fluoro-6-methyl-1-[1-(tetrahydro-2H-pyran-4-yl)-4-piperidinyl]-1,3-dihydro-2H-benzimidazol-2-one and pharmaceutically acceptable salts and solvates thereof, described in WO 2007/036715;
- 5-Fluoro-6-methyl-1-[1-(tetrahydro-2H-pyran-4-yl)-4-piperidinyl]-1,3-dihydro-2H-benzimidazol-2-one and pharmaceutically acceptable salts and solvates thereof, described in WO2007/036718 and US 8,288,412;
- 4-(*R*)-ethyl-3-(2-methylbenzamido)-1,4'-bipiperidine-1'-carboxylate and pharmaceutically acceptable salts and solvates thereof, described in WO 2010/096703;
- ethyl 3-[(3-exo)-(2-benzamidoethyl)amino]-8-azabicyclo[3.2.1]octane-8-carboxylate and pharmaceutically acceptable salts and solvates thereof, described in US 8,697691;
- 5-(3-ethyl-1,2,4-oxadiazol-5-yl)-1,4,5,6-tetrahydropyrimidine (MCD-386) and pharmaceutically acceptable salts and solvates thereof, described in US 8,853,219;
- 3-Methyl-5-(piperidin-3-yl)-1,2,4-oxadiazole, its enantiomers and pharmaceutically acceptable salts and solvates thereof, described in US 8,853,219; and
- 3-[(1S,2S)-2-hydroxycyclohexyl]-6-[(6-methylpyridin-3-yl)methyl]benzo[h] quinazolin-4(3H)-one (MK-7622), described in US 8,883,810 and pharmaceutically acceptable salts and solvates thereof, especially the fumarate, the methanesulfonate or the hydrochloride.

The amount of the MCRA Component (a) of the combination, i.e. a single MCRA dose, may vary according to intrinsic muscarinic cholinergic receptor potency of said component and is present in an amount from 1-times to up to six times the maximum amount contained in the commercial products or of the maximal, single MCRA dose administered during the clinical trials of each MCRA, that is considered to be equivalent to the Maximum Tolerated Dose as determined during the clinical trials. Advantageously, said dose is from 1-time to 6 times, advantageously from 1.2-times to 6-times, normally from 1.2 times to 4 times higher than the maximum amount contained in the commercial products at the time of filing or of the maximal, single MCRA dose administered during the clinical trials.

In general, and particularly when data from commercial products or clinical trials is not available, for an individual patient, the MCRA Component (a) in the combination of the present invention is present in an amount from 1-times to up to 6-times greater than the amount of MCRA Component (a) that first induces vomiting and/or diarrhea in said patient when the MCRA Component (a) is administered alone.

For most patients, the above criteria will result in the MCRA Component (a) in the combination of the present invention being present in an amount from 0.5 mg to 1500 mg.

In particular, the maximum dose/unit form approved for cevimeline (as hydrochloride hemihydrate) is 30 mg, to be administered three times per day.

However, as set forth above, cevimeline is the sole MCRA approved for a pharmacological treatment, said treatment having no relation with any form of CNS disorder such as dementia.

Among the other MCRAs tested in clinical trials for the treatment of Alzheimer disease, in view of the aforementioned Mirza et al 2003 (CNS Drug Reviews Vol. 9, No. 2, pp. 159-186) paper showing that, in the context of the present invention the maximum tolerated dose of said xanomeline is considered as being 75 mg three times per day, i.e. 225 mg/day.

Thus, for example, in the combination of the present invention cevimeline, as hydrochloride hemihydrate, is present in an amount of from 30 mg to 180 mg, advantageously from more than 30 mg to 180 mg, preferably from 36 mg to 180 mg, normally from 30 mg to 120 mg; milameline, as hydrochloride, is present in an amount of from 2 mg to 12 mg, advantageously from more than 2 mg to 12 mg, preferably from 2.4 mg to 12 mg, normally from 2.4 mg to 8 mg; xanomeline, as free base, as oxalate or as L-tartrate, is present in an amount of from 75 mg to 450 mg, advantageously from more than 75 mg to 450 mg. preferably from 90 mg to 450 mg, normally from 90 mg to 180 mg; and MK-7622, especially as hydrochloride, methanesulfonate or fumarate, is present in an amount of from 5 mg to 270 mg, advantageously from more than 5 mg to 270 mg, preferably from 6 mg to 270 mg, normally from 15 mg to 225 mg. For the administration for the treatment of a hypocholinergic disorder in a patient, each MCRA is formulated in a pharmaceutical composition in dosage unit form in admixture with a pharmaceutical carrier or vehicle.

In the combination of the present invention, xanomeline may be present in a pharmaceutical composition in dosage unit form, in admixture with a pharmaceutical carrier or vehicle in a TTS formulation, in particular in a patch for transdermal administration. Advantageously, xanomeline is released from a patch in an amount giving xanomeline plasma concentrations of from 15.572ng/ml to 78.6ng/ml.

According to the present invention, the safe daily dose of said MCRA may be 3-6 times higher than the dose of MCRA alone that caused vomiting in a particular human being, especially in a patient suffering from a hypocholinergic disorder, or up to six times the average maximal tolerated dose of said MCRA as determined in clinical trials when used alone, due to the competitive action of the MCRA/ naAEA combination. Normally, it is from 1 times to 6 times, advantageously from more than 1 times to 6 times, preferably from 1.2 times to 6 times, normally from 1.2 times to 4 times said maximal tolerated MCRA dose or from 1 times to 6 times, advantageously from more than one times to 6 times, preferably from 1.2 times to 6 times, normally from 1.2 to 4 times the maximal daily dose of each MCRA, as previously administered alone to patients during the respective clinical trials.

In particular, using the combination of the present invention, the daily dose of cevimeline, as hydrochloride hemihydrate, is in general from 90 mg to 540 mg, advantageously from more than 90 mg to 540 mg, preferably from 108 mg to 540 mg, normally from 108 mg to 360 mg; the daily dose of milameline, as hydrochloride, in general is from 8 mg to 48 mg, advantageously from more than 8 mg to 48 mg, preferably from 9.6 mg to 48 mg, normally from 9.6 mg to 32 mg; the daily dose of xanomeline, as oxalate or L-tartrate, in general is from 300 mg to 1350 mg, advantageously from more than 300 mg to 1350 mg preferably from 337.5 mg to 1350 mg, normally from 337.5 mg to 900 mg; and the daily dose of MK-7622, as hydrochloride, fumarate or methanesulfonate, in general, is from 5 mg to 270 mg, advantageously from more than 5 mg to 270 mg, preferably from 6 mg to 270 mg, normally from 15 mg to 225 mg.

### The naAEAs Component (b)

Antiemetic medications commonly used to treat emesis, and not primarily regarded as anticholinergic agents, that are entirely devoid of anticholinergic activity or have an extremely low ability to prevent acetylcholine from acting at its cholinergic receptor sites in the brain may be used as Component (b) of the pharmaceutical combination of the present invention.

Preferably, said Component (b) is a non-anticholinergic antiemetic agent selected from the group consisting of (b1) 5HT3-antagonists, (b2) DA-antagonists, (b3) HI-antagonists, (b4) cannabinoids, and (b5) NK1-antagonists.

Typical non-anticholinergic antiemetic agents are
- 5-HT3 receptor antagonists (5HT3-antagonists), such as 9-methyl-3-[(2-methyl-1H-imidazol-1-yl)methyl]-1,2,3,9-tetrahydrocarbazol-4-one (ondansetron) and pharmaceutically acceptable salts and solvates thereof, in particular its hydrochloride dihydrate, described in EP 191562; 3S-ondansetron; 3R-onsdansetron; (3R)-10-oxo-8-azatricyclo[5.3.1.03,8]undec-5-yl 1H-indole-3-carboxylate (dolasetron) and pharmaceutically acceptable salts and solvates thereof, in particular its monomethanesulfonate (mesylate or mesilate) monohydrate, described in EP 266730; 1-methyl-N-(9-methyl-9-azabicyclo[3.3.1]non-3-yl)-indazole-3-carboxamide (granisetron) and pharmaceutically acceptable salts and solvates thereof, in particular its hydrochloride, described in EP 200444; [(1S,5S)-8-methyl-8-azabicyclo[3.2.1]oct-3-yl]1H-indole-3-carboxylate (tropisetron) and pharmaceutically acceptable salts and solvates thereof, in particular its monohydrochloride, described in U.S. Pat. No. 4,789,673; 1-phenylmethyl-2-piperazinyl-1H-benzimidazole (lerisetron) and pharmaceutically acceptable salts and solvates thereof, in particular its hydrochloride, described in EP 512939; (R)-5-[(1-methyl-3-indolyl)carbonyl]-4,5,6,7-tetrahydro-1H-benzimidazole (ramosetron) and pharmaceutically acceptable salts and solvates thereof, in particular its hydrochloride, described in U.S. Pat. No. 5,344,927; (3aR)-2-[(3S)-1-azabicyclo[2.2.2]oct-3-yl]-2,3,3a,4,5,6-hexahydro-1H-benz[de]isoquinolin-1-one (palonosetron) and pharmaceutically acceptable salts and solvates thereof, in particular its hydrochloride, described in U.S. Pat. No. 5,202,333; 2,3,4,5-tetrahydro-5-methyl-2-[(5-methyl-1H-imidazol-4-yl)methyl]-1H-pyrido[4,3-b]indol-1-one (alosetron) and pharmaceutically acceptable salts and solvates thereof, in particular its hydrochloride, described in U.S. Pat. No. 5,360,800; and (±)-6-chloro-,3,4-dihydro-4-methyl-3-oxo-N-(quinuclidinyl)-2H-1,4-benzoxazine-8-carboxamide (azasetron) and pharmaceutically acceptable salts and solvates thereof, in particular its hydrochloride, described in U.S. Pat. No. 4,892,872; which are known to be serotonin receptors blockers in the central nervous system and gastrointestinal tract and have been proposed for use to treat post-operative and cytotoxic drug nausea and vomiting;
- dopamine antagonists ("DA-antagonists"), such as 5-chloro-1-(1-[3-(2-oxo-2,3-dihydro-1H-benzo[d]imidazol-1-yl)propyl]piperidin-4-yl)-1H-benzo[d]imidazol-2(3H)-one (domperidone) and pharmaceutically acceptable salts and solvates thereof, particularly its maleate; 1-[1-[4-(4-fluorophenyl)-4-oxo-butyl]-3,6-dihydro-2H-pyridin-4-yl]-3H-benzoimidazol-2-one (droperidol); 4-[4-(4-chlorophenyl)-4-hydroxy-1-piperidyl]-1-(4-fluorophenyl)-butan-1-one (haloperidol); 4-aminosalicylamide and benzamide derivatives like 4-amino-5-chloro-N-[2-(diethylamino)ethyl]-2-methoxybenzamide (metoclopramide) and pharmaceutically acceptable salts and solvates thereof such as its monohydrochloride monohydrate; 4-amino-5-bromo-N-[2-(diethylamino)ethyl]-2-methoxybenzamide (bromopride) and pharmaceutically acceptable salts and solvates thereof, particularly its monohydrochloride and its dihydrochloride monohydrate; 4-amino-N-(1-benzylpiperidin-4-yl)-5-chloro-2-methoxybenzamide (clebopride) and pharmaceutically acceptable salts and solvates thereof, particularly its malate or its hydrochloride monohydrate; N-[(1-allylpyrrolidin-2-yl)methyl]-6-methoxy-1H-benzo[d][1,2,3]triazole-5-carboxamide (alizapride) and pharmaceutically acceptable salts and solvates thereof, particularly its hydrochloride; (L)-2-methoxy-N-((1-propylpyrrolidin-2-yl)methyl)-5-sulfamoylbenzamide (levosulpiride); N-{[4-(2-dimethylaminoethoxy)phenyl]methyl}-3,4,5-trimethoxy-benzamide (trimethobenzamide) and pharmaceutically acceptable salts and solvates thereof, particularly its hydrochloride;
   which act in the brain and especially at the chemoreceptor trigger zone and are known to be used to treat nausea and vomiting associated with neoplastic disease, radiation sickness, opioids, cytotoxic drugs and general anesthetics;
- H1 histamine receptor antagonists ("HI-antagonists"), such as 1-[(4-chlorophenyl)-phenyl-methyl]-4-[(3-methylphenyl)methyl]piperazine (meclizine or meclozine) and pharmaceutically acceptable salts and solvates thereof, particularly its dihydrochloride monohydrate; dimethyl[1-(10H-phenothiazin-10-yl)propan-2-yl]amine (promethazine) and pharmaceutically acceptable salts and solvates thereof, particularly its hydrochloride; 3-(2-chloro-10H-phenothiazin-10-yl)-N,N-dimethyl-propan-1-amine (chlorpromazine) or a salt thereof, particularly its hydrochloride; 2-chloro-10-[3-(4-methyl-1-piperazinyl)propyl]-10H-phenothiazine (prochlorperazine) and pharmaceutically acceptable salts and solvates thereof, particularly its dimaleate, dimesylate or 1,2-ethanedisulfonate (1:1) (edisilate); and 2-(2-{4-[(4-chlorophenyl)(phenyl)methyl]piperazin-1-yl}ethoxy)ethanol (hydroxyzine) and pharmaceutically acceptable salts and solvates thereof such as its hydrochloride or 1,1'-methylene-bis(2-hydroxy-3-naphthalenecarboxylic acid salt (pamoate),
   which are known to be effective in many conditions, including motion sickness and severe morning sickness in pregnancy;
- cannabinoid receptor agonists ("cannabinoids"), such as cannabis; (6aR-trans)-6a,7,8,10a-tetrahydro-6,6,9-trimethyl-3-pentyl-6H-dibenzo[b,d]pyran-1-ol (dronabinol); (6aR,10aR)-rel-3-(1,1-dimethylheptyl)-6,6a,7,8,10,10a-hexahydro-1-hydroxy,6,6-dimethyl-9H-dibenzo[b,d]pyran-9-one (nabilone); and (-)-cis-3-[2-hydroxy-4-(1,1-dimethylheptyl)-phenyl]-trans-4-(3-hydroxypropyl)cyclohexanol (CP 55,940);
   which are known to be used in patients with cachexia and cytotoxic nausea and vomiting; and
- antagonists of the neurokinin 1 receptor (NK1-antagonists) such as 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (aprepitant); (2S,4S)-4-(4-Acetyl-1-piperazinyl)-N-[(1R)-1-[3,5-bis(trifluoromethyl)phenyl]ethyl]-2-(4-fluoro-2-methylphenyl)-N-methyl-1-piperidinecarboxamide (casopitant); 2-[3,5-bis(trifluoromethyl)phenyl]-*N*,2-dimethyl-*N*-[4-(2-methylphenyl)-6-(4-methyl-1-piperazinyl)-3-pyridinyl]propanamide (netupitant) described in US 6,297,375, US 6,719,996 and US 6,593,47; the disclosures of which are incorporated herein in their entirety; and (5*S*,8*S*)-8-({(1*R*)-1-[3,5-bis(trifluoromethyl)phenyl]ethoxy}methyl)-8-phenyl-1,7-diazaspiro[4.5]decan-2-one (rolapitant), described in US 7,049,320 and, for an injectable form thereof, in US 9,101,615; the disclosures of which are incorporated herein in their entireties; which are known to be neurokinine-1 receptors blockers in both the central and peripheral nervous system and have been proposed for use to treat cytotoxic drug nausea and vomiting.

Advantageous naAEAs are the compounds available in drugs for current antiemetic therapy, in particular,
- alosetron hydrochloride, available at the oral dose/unit form (in alosetron) of 0.5-1 mg;
- azasetron hydrochloride or mesilate monohydrate, available at the oral or i.v. dose/unit form of 10 mg;
- dolasetron mesylate monohydrate, available at the oral dose/unit form (in dolasetron) of 50-100 mg;
- granisetron hydrochloride, available at the oral dose (in granisetron) of 1-2 mg;
- ondansetron hydrochloride dihydrate, available at the oral dose (in ondansetron) of 4-8 mg;
- palonosetron hydrochloride, available at a the oral dose (in palonosetron) of 0.5 mg and i.v. dose (in palonosetron) of 0.25 mg;
- tropisetron hydrochloride, available at the oral dose (in tropisetron) of 5 mg;
- domperidone, available at the dose of 10 mg;
- haloperidol, available at the oral dose of 1-10 mg;
- chlorpromazine hydrochloride, available at the oral dose (in chlorpromazine) of 25-100 mg;
- prochlorperazine dimaleate, available at the oral dose of 5 mg;
- metoclopramide hydrochloride dihydrate, available at the oral dose (in metoclopramide) of 10 mg;
- bromopride dihydrochloride monohydrate, available at the oral dose (in bromopride) of 10 mg;
- clebopride malate (1:1), available at a oral dose (in clebopride) of 1 mg;
- levosulpiride, at the oral dose of 25-100 mg;
- alizapride hydrochloride, available at the oral dose (in alizapride) of 50 mg;
- trimethobenzamide hydrochloride, available at the oral dose (in trimethobenzamide) of 100 mg
- meclizine (also called meclozine), available at the oral dose of 12.5-50 mg;
- promethazine hydrochloride, available at the oral dose (in promethazine) of 25 mg;
- dronabinol, available at the oral dose of 0.5-1 mg;
- aprepitant, available at the oral dose of 40-125 mg;
- netupitant, available at the oral dose of 300 mg; and
- casopitant, at the oral dose of 50 mg;
- rolapitant, available at the oral dose of 60 mg;
- the palonosetron-0.5 mg/netupitant-300 mg oral fixed-dose combination.

In the pharmaceutical combination to improve the treatment of human dementias of the Alzheimer type according to the present invention, the non-anticholinergic antiemetic agent Component (b) is formulated in a pharmaceutical composition in admixture with a pharmaceutical carrier to be administered in combination with the MCRA Component (a).

The amount of the MCRA Component (a), sufficient to maximally alleviate disease-associated cognitive and other neurobehavioral symptoms; is illustrated in the above "The MCRAs" section.

The Component (b) is advantageously selected from the group consisting of alosetron and pharmaceutically acceptable salts and solvates thereof, especially its hydrochloride; azasetron and pharmaceutically acceptable salts and solvates thereof, especially its hydrochloride; ondansetron and pharmaceutically acceptable salts and solvates thereof, especially its monohydrochloride dihydrate; granisetron and pharmaceutically acceptable salts and solvates thereof, especially its hydrochloride; dolasetron and pharmaceutically acceptable salts and solvates thereof, especially its monomethanesulfonate monohydrate, ramosetron and pharmaceutically acceptable salts and solvates thereof, especially its hydrochloride; tropisetron and pharmaceutically acceptable salts and solvates thereof, especially its hydrochloride; palonosetron and pharmaceutically acceptable salts and solvates thereof, especially its hydrochloride; domperidone and pharmaceutically acceptable salts and solvates thereof, especially its maleate; metoclopramide and pharmaceutically acceptable salts and solvates thereof, especially its hydrochloride monohydrate; bromopride and pharmaceutically acceptable salts and solvates thereof, especially its monohydrochloride or the dihydrochloride monohydrate; alizapride and pharmaceutically acceptable salts and solvates thereof, especially its hydrochloride; clebopride and pharmaceutically acceptable salts and solvates thereof, especially its malate and the hydrochloride monohydrate; meclizine (meclozine) and pharmaceutically acceptable salts and solvates thereof, especially its hydrochloride monohydrate; promethazine and pharmaceutically acceptable salts and solvates thereof, especially its hydrochloride; chlorpromazine and pharmaceutically acceptable salts and solvates thereof, especially its hydrochloride; prochlorperazine and pharmaceutically acceptable salts and solvates thereof, especially its dimaleate, its dimesylate and its the 1,2-ethanedisulfonate (1:1) (edisilate); hydroxyzine and pharmaceutically acceptable salts and solvates thereof such as the dihydrochloride or the 1,1'-methylene bis(2-hydroxy-3-naphthalenecarboxylic acid (pamoate); dronabinol; nabilone; aprepitant; netupitant; rolapitant; and casopitant.

In the combination of the present invention, the non-anticholinergic antiemetic agent Component (b) is present in an amount of from 50% to 600%, normally 50% to 300%, of the amount of the said non-anticholinergic antiemetic agent contained as a sole active ingredient in the currently used brand or generic drugs. Each of said typical non-anticholinergic antiemetic agents is present, in admixture with a pharmaceutical carrier or vehicle, in a pharmaceutical composition in dosage unit form, as Component (b), in an amount ranging from 50% of the minimum amount to 600%, and in some cases beyond 600%, advantageously from 50% to 300%, normally from 100% to 300%, of the maximum amount of said typical non-anticholinergic antiemetic agent contained in the corresponding, currently used generic or brand drug for its antiemetic indication in IR form. Advantageously, the currently used brand or generic drugs containing the maximum amount of said naAEA may be used as Component (b) of the combination of the present invention.

Advantageous naAEAs in said Component (b) are selected from the group consisting of alosetron and pharmaceutically acceptable salts and solvates thereof, in particular the hydrochloride, in an amount (in alosetron) of from 0.25 mg to 6 mg; azasetron and pharmaceutically acceptable salts and solvates thereof, in particular the hydrochloride, in an amount (in azasetron) of from 5 mg to 60 mg; dolasetron and pharmaceutically acceptable salts and solvates thereof, in particular the mesylate, in an amount (in dolasetron) of from 25 mg to 600 mg; granisetron and pharmaceutically acceptable salts and solvates thereof, in particular the hydrochloride, in an amount (in granisetron) of from 0.5 mg to 6 mg; ondansetron and pharmaceutically acceptable salts and solvates thereof, in particular the hydrochloride dihydrate, in an amount (in ondansetron) of from 2 mg to 64 mg; palonosetron and pharmaceutically acceptable salts and solvated thereof, in particular its hydrochloride, in an amount (in palonosetron) of from 0.25 mg to 3 mg; ramosetron and pharmaceutically acceptable salts and solvates thereof, in particular its hydrochloride, in an amount (in ramosetron) of from 0.0125 mg to 0.3 mg; tropisetron and pharmaceutically acceptable salts and solvates thereof, in particular the hydrochloride, in an amount of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine and pharmaceutically acceptable salts and solvates thereof, in particular the hydrochloride, in an amount (in chlorpromazine) of from 12.5 mg to 600 mg; prochlorperazine and pharmaceutically acceptable salts and solvates thereof, in particular the dimaleate, in an amount (in prochlorperazine) of from 2.5 mg to 30 mg; metoclopramide and pharmaceutically acceptable salts and solvates thereof, in particular the monohydrochloride monohydrate, in an amount (in metoclopramide) of from 5 mg to 60 mg; bromopride and pharmaceutically acceptable salts and solvates, in particular the monohydrochloride and the dihydrochloride monohydrate, in an amount (in bromopride) of from 5 mg to 60 mg; clebopride and pharmaceutically acceptable salts and solvates thereof, in particular the hydrogen malate and the hydrochloride monohydrate, in an amount (in clebopride) of from 0.25 mg to 3 mg; levosulpiride, in an amount of from 12.5 mg to 600 mg; alizapride and pharmaceutically acceptable salts thereof, in particular the hydrochloride, in an amount (in alizapride) of from 25 mg to 300 mg; trimethobenzamide and pharmaceutically acceptable salts thereof such as the monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine and pharmaceutically acceptable salts and solvates thereof, in particular the hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

According to an embodiment, the non-anticholinergic antiemetic is present, in an IR unit form, in an amount ranging from 50% to 200% of the maximum amount of said antiemetic agent contained in the currently administered IR dosage unit forms for the treatment of the above-cited disorders or, in an ER unit form, in an amount ranging from 75% to 300% of the maximum amount of said antiemetic agent contained in the currently administered IR dosage unit forms for the treatment of the above-cited disorders.

For example, according to this embodiment, among the advantageous non-anticholinergic antiemetic agents used as Component (b), in said composition ondansetron or a pharmaceutically acceptable salt or solvate thereof, in particular its hydrochloride dihydrate, is present in an amount (in ondansetron) of from 4 mg to 16 mg per dosage unit in an IR unit form or in an amount of from 6 mg to 48 mg, preferably from 16 mg to 32 mg, in an ER unit form; alosetron or a pharmaceutically acceptable salt thereof, in particular its hydrochloride, is present in an amount (in alosetron) of from 0.5 mg to 2 mg per dosage unit in an IR unit form or in an amount of from 0.75 mg to 3 mg, in an ER unit form; azasetron or a pharmaceutically acceptable salt thereof, in particular its hydrochloride, is present in an amount of from 5 mg to 20 mg per dosage unit in an IR unit form or in an amount of from 7.5 mg to 30 mg, preferably from 10 mg to 30 mg, in an ER unit form; ramosetron or a pharmaceutically acceptable salts thereof, in particular its hydrochloride, is present in an amount (in ramosetron) of from 0.025 mg to 0.1 mg per dosage unit in an IR unit form or in an amount of from 0.0375 mg to 0.3 mg, preferably from 0.05 mg to 0.3 mg, in an ER unit form; tropisetron or a pharmaceutically acceptable salt thereof, in particular its hydrochloride, is present in an amount (in tropisetron) of from 2.5 mg to 10 mg per dosage unit in an IR unit form or in an amount of from 3.75 mg to 15 mg, preferably from 5 mg to 15 mg, in an ER unit form; granisetron or a pharmaceutically acceptable salt thereof, in particular its hydrochloride, is present in an amount (in granisetron) of from 1 mg to 4 mg per dosage unit in an IR unit form or in an amount of from 1.5 mg to 6 mg, in an ER unit form; dolasetron, or a pharmaceutically acceptable salt thereof, in particular its mesilate, is present in an amount (in dolasetron) of from 50 mg to 200 mg per dosage unit in an IR unit form or in an amount of from 75 mg to 300 mg, in an ER unit form; palonosetron, or a pharmaceutically acceptable salt thereof, in particular its hydrochloride, is present (a) in an amount (in palonosetron) of from 0.25 mg to 1 mg in an IR dosage unit form or from 0.375 mg to 1.5 mg in an ER dosage unit form, or (b) in an amount (in palonosetron) of from 0.25 mg to 12 mg, in a fixed-dose combination with netupitant, in an amount of from 200 mg to 600 mg, said fixed-dose combination being in an IR dosage unit form; domperidone or a pharmaceutically acceptable salt thereof, in particular its maleate, is present in an amount (in domperidone) of from 5 mg to 20 mg per dosage unit in an IR unit form or in an amount of from 7.5 mg to 30 mg, preferably from 10 mg to 30 mg, in an ER unit form; metoclopramide or a pharmaceutically acceptable salt or solvate thereof, in particular its monohydrochloride monohydrate, is present in an amount (in metoclopramide) of from 5 mg to 20 mg per dosage unit in an IR unit form or in an amount of from 7.5 mg to 30 mg, preferably from 10 mg to 30 mg, in an ER unit form; alizapride or a pharmaceutically acceptable salt thereof, in particular its hydrochloride, is present in an amount (in alizapride) of from 25 mg to 100 mg per dosage unit in an IR unit form or in an amount of from 37.5 mg to 300 mg, preferably from 100 mg to 300 mg, in an ER unit form; meclizine or a pharmaceutically acceptable salt thereof, in particular its hydrochloride is present in an amount (in meclizine) of from 25 mg to 100 mg per dosage unit in an IR unit form or in an amount of from 37.5 mg to 150 mg, preferably from 50 mg to 150 mg, in an ER unit form; chlorpromazine or a pharmaceutically acceptable salt thereof, in particular its hydrochloride is present in an amount (in chlorpromazine) of from 50 mg to 200 mg per dosage unit in an IR unit form or in an amount of from 75 mg to 300 mg, preferably from 100 mg to 300 mg, in an ER unit form; prochlorperazine or a pharmaceutically acceptable salt thereof, in particular its maleate is present in an amount (in prochlorperazine) of from 2.5 mg to 10 mg per dosage unit in an IR unit form or in an amount of from 3.75 mg to 15 mg, preferably from 5 mg to 15 mg, in an ER unit form; dronabinol is present in an amount of from 5 mg to 20 mg per dosage unit in an IR unit form or in an amount of from 7.5 mg to 30 mg, preferably from 10 mg to 30mg, in an ER unit form; nabilone is present in an amount of from 0.5 mg to 2 mg per dosage unit in an IR unit form or in an amount of from 0.75 mg to 3 mg per dosage unit in an ER unit form; aprepitant is present in an amount of from 62.5 mg to 250 mg per dosage unit in an IR unit form or in an amount of from 93.75 mg to 325 mg, preferably from 125 mg to 325 mg, in an ER unit form; netupitant is present in an amount of from 150 mg to 600 mg, in an IR unit form or in an amount of from 225 to 900 mg, preferably from 300 mg to 900 mg, in an ER unit form; rolapitant, in an amount of form 30 mg to 120 mg, in an IR unit form or in an amount of from 45 mg to 180 mg, preferably from 60 mg to 180 mg, in an ER unit form; and casopitant is present in an amount of from 25 mg to 100 mg per dosage unit in an IR unit form or in an amount of from 37.5 mg to 150, preferably from 50 mg to 150 mg, in an ER unit form, in admixture with a pharmaceutical composition in dosage unit form.

Preferred Component (b) is a pharmaceutical composition in dosage unit form comprising a non-anticholinergic antiemetic agent selected from the group consisting of ondansetron and pharmaceutically acceptable salts and solvates thereof, in an amount (in ondansetron) of from 8 mg to 24 mg; granisetron and pharmaceutically acceptable salts and solvates thereof, in an amount (in granisetron) of from 1 mg to 3 mg; domperidone and pharmaceutically acceptable salts and solvates thereof, in an amount (in domperidone) of from 10 mg to 30 mg; metoclopramide and pharmaceutically acceptable salts and solvates thereof, in an amount (in metoclopramide) of from 10 mg to 30 mg; dronabinol, in an amount of from 10 mg to 30 mg; nabilone, in an amount of from 1 mg to 3 mg; aprepitant, in an amount of from 125 mg to 375 mg; netupitant, in an amount of from 300 mg to 900 mg; rolapitant, in an amount of form 60 mg to 180 mg; and casopitant, in an amount of from 50 mg to 150 mg, in admixture with a pharmaceutical carrier.

Thus, the invention provides compositions and methods for treating hypocholinergic disorders, which comprises administering to a patient in need of said treatment the above-illustrated combination. In such a treatment, Component (a) and Component (b) of the combination may be administered simultaneously or sequentially to said patient, Component (a) being indifferently administered before or after Component (b). Component (a) and Component (b) may also be administered by the same or a different administration route.

The invention may also include a third component, Component (c), that is an AChEI, also formulated in a pharmaceutical composition.

### The Combinations

The present invention provides the combination of any MCRA and any naAEA as exemplified in the respective sections herein, each formulated in pharmaceutical composition in admixture with a pharmaceutical carrier.

In particular, the combination of the present invention may be a combination comprising or consisting essentially of
(a) any of the MCRAs such as those described hereinabove, each in a pharmaceutical composition in dosage unit form, in admixture with a pharmaceutical carrier, said MCRA being preferably selected from the group consisting ofAF267 and pharmaceutically acceptable salts and solvates thereof; cevimeline and pharmaceutically acceptable salts and solvates thereof; EUK 1001 and pharmaceutically acceptable salts and solvates thereof; milameline and pharmaceutically acceptable salts and solvates thereof; RS-86 and pharmaceutically acceptable salts and solvates thereof; sabcomeline and pharmaceutically acceptable salts and solvates thereof; talsaclidine and pharmaceutically acceptable salts and solvates thereof; tazomeline and pharmaceutically acceptable salts and solvates thereof; xanomeline and pharmaceutically acceptable salts and solvates thereof; AC-42 and pharmaceutically acceptable salts and solvates thereof; TBPB and pharmaceutically acceptable salts and solvates thereof; 4-Fluoro-6-methyl-1-[1-(tetrahydro-2H-pyran-4-yl)-4-piperidinyl]-1,3-dihydro-2H-benzimidazol-2-one; and pharmaceutically acceptable salts and solvates thereof;5-Fluoro-6-methyl-1-[1-(tetrahydro-2H-pyran-4-yl)-4-piperidinyl]-1,3-dihydro-2H-benzimidazol-2-one and pharmaceutically acceptable salts and solvates thereof;4-(R)-ethyl-3-(2-methylbenzamido)-1,4'-bipiperidine-1'-carboxylateand pharmaceutically acceptable salts and solvates thereof; ethyl 3-[(3-exo)-(2-benzamidoethyl)amino]-8-azabicyclo[3.2.1]octane-8-carboxylate and pharmaceutically acceptable salts and solvates thereof; 5-(3-ethyl-1,2,4-oxadiazol-5-yl)-1,4,5,6-tetrahydropyrimidine (MCD-386) and pharmaceutically acceptable salts and solvates thereof; 3-Methyl-5-(piperidin-3-yl)-1,2,4-oxadiazole, its enantiomers and pharmaceutically acceptable salts and solvates thereof; and MK-7622 and pharmaceutically acceptable salts and solvates thereof; and
(b) any of the naAEA such as those described herein, each in a pharmaceutical composition in admixture with a pharmaceutical carrier, said naAEA being preferably selected from the group consisting of alosetron and pharmaceutically acceptable salts and solvates thereof; dolasetron and pharmaceutically acceptable salts and solvates thereof; granisetron and pharmaceutically acceptable salts and solvates thereof; ondansetron and pharmaceutically acceptable salts and solvates thereof; palonosetron and pharmaceutically acceptable salts and solvates thereof; domperidone and pharmaceutically acceptable salts and solvates thereof; tropisetron and pharmaceutically acceptable salts and solvates thereof; haloperidol; chlorpromazine and pharmaceutically acceptable salts and solvates thereof; prochlorperazine and pharmaceutically acceptable salts and solvates thereof; metoclopramide and pharmaceutically acceptable salts and solvates thereof; bromopride and pharmaceutically acceptable salts and solvates thereof; clebopride and pharmaceutically acceptable salts and solvates thereof; levosulpiride; alizapride and pharmaceutically acceptable salts thereof; trimethobenzamide and pharmaceutically acceptable salts thereof; meclizine (also called meclozine) and pharmaceutically acceptable salts and solvates thereof; promethazine and pharmaceutically acceptable salts and solvates thereof; dronabinol; nabilone; aprepitant; netupitant; rolapitant; casopitant.

In the above combination, each of the Components (a) and (b) is in pharmaceutical composition in dosage unit form wherein each of said components is in admixture with a pharmaceutical carrier or vehicle.

A particularly advantageous combination essentially consists of
(a) a MCRA selected from the group consisting of cevimeline and pharmaceutically acceptable salts thereof, in an amount (in cevimeline) of from 34.5 mg to 180 mg; milameline and pharmaceutically acceptable salts thereof, in an amount (in milameline) of from 2.4 mg to 12 mg; xanomeline and pharmaceutically acceptable salts thereof, in an amount (in xanomeline) of from 90 mg to 450 mg; and MK-7622 and pharmaceutically acceptable salts thereof, in an amount (in MK-7622) of from 5 mg to 270 mg; in a pharmaceutical composition in admixture with a pharmaceutical carrier; and
(b) a naAEA selected from the group consisting of ondansetron and pharmaceutically acceptable salts and solvates thereof, in an amount (in ondansetron) of fro 4 mg to 64 mg, domperidone and pharmaceutically acceptable salts and solvates thereof, in an amount, in domperidone of from 5 mg to 30 mg; and metoclopramide and pharmaceutically acceptable salts and solvates the, in an amount (in metoclopramide) of from 5 mg to 30 mg; in a pharmaceutical composition in admixture with a pharmaceutical carrier.

According to a first embodiment, an advantageous combination may be a combination comprising or consisting essentially of
(a) a MCRA selected from the group consisting of cevimeline, cevimeline hydrochloride hemihydrate, milameline, milameline hydrochloride, xanomeline, xanomeline oxalate, xanomeline L-tartrate, racemic 3-Methyl-5-(piperidin-3-yl)-1,2,4-oxadiazole and pharmaceutically acceptable salts and solvates thereof, S-(+)-3-methyl-5-(piperidin-3-yl)-1,2,4-oxadiazole D-tartrate; R-(-)-3-Methyl-5-(piperidin-3-yl)-1,2,4-oxadiazole L-tartrate; MK-7622, MK-7622 hydrochloride, MK-7622 methanesulfonate and MK-7622 fumarate, in a pharmaceutical composition in admixture with a pharmaceutical carrier or vehicle; and
(b) a naAEA, in a pharmaceutical composition in admixture with a pharmaceutical carrier or vehicle.

According to this first embodiment, a preferred combination may be a combination comprising or consisting essentially of
(a) a MCRA selected from the group consisting of cevimeline hydrochloride hemihydrate, in an amount of from 34.5 mg to 180 mg; xanomeline, as free base, as oxalate or as L-tartrate, in an amount of from 90 mg to 450 mg; milameline hydrochloride, in an amount of from 2.4 mg to 12 mg; and MK 7622, as free base, as hydrochloride, as fumarate or as methanesulfonate, in an amount consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in a pharmaceutical composition in admixture with a pharmaceutical carrier or vehicle; and
(b) a naAEA in a pharmaceutical composition in admixture with a pharmaceutical carrier or vehicle.

Preferably, in this combination, cevimeline, Component (a), as free base or hydrochloride hemihydrate, is present in an amount of from 36 mg to 180 mg; and MK-7622, as free base, as hydrochloride, as methanesulfonate or as fumarate, is present in an amount consisting of from 5 mg to 270 mg, in particular of from more than 5 mg to 15 mg, from more than 15 mg to 45 mg, from more than 45 mg to 270 mg, from more than 15 mg to 225 mg, from more than 45 mg to 225 mg, and from 54 mg to 180 mg.

The pharmaceutical combinations of this first embodiment of the present invention, are useful for the treatment of hypocholinergic disorders, and even high doses of a MCRA Component (a), may be present to improve symptoms without adverse effects to a greater extent.

Thus, the present invention provides a method for treating hypocholinergic disorders, which comprises administering to a patient in need of said treatment the combinations described herein in one embodiment. In such a treatment, Component (a), and Component (b) of the combination may be administered simultaneously or sequentially to said patient, Component (a) being indifferently administered before or after Component (b). Component (a) and Component (b) may also be administered by the same or a different administration route.

According to a second embodiment, the present invention provides a pharmaceutical combination comprising or consisting essentially of, as Components:
(a) a MCRA, in a pharmaceutical composition in admixture with a pharmaceutical carrier or vehicle; and
(b) a naAEA selected from the group consisting of (b1) 5HT3-antagonists, (b2) DA-antagonists, (b3) HI-antagonists, (b4) cannabinoids, (b5) NK1-antagonists, and the netupitant-palonosetron fixed-dose combination, in a pharmaceutical composition in admixture with a pharmaceutical carrier or vehicle.

Another advantageous combination is one comprising or consisting essentially of
(a) a MCRA, in a pharmaceutical composition in admixture with a pharmaceutical carrier or vehicle; and
(b) a naAEA consisting of a 5HT3-antagonist selected from the group consisting of alosetron and pharmaceutically acceptable salts and solvates thereof, azasetron and pharmaceutically acceptable salts and solvates thereof; ondansetron and pharmaceutically acceptable salts and solvates thereof; granisetron and pharmaceutically acceptable salts and solvates thereof; dolasetron and pharmaceutically acceptable salts and solvates thereof, ramosetron and pharmaceutically acceptable salts and solvates thereof; tropisetron and pharmaceutically acceptable salts and solvates thereof; and palonosetron and pharmaceutically acceptable salts and solvates thereof, in a pharmaceutical composition in admixture with a pharmaceutical carrier or vehicle.

According to this second embodiment, another advantageous combination is a combination comprising or consisting essentially of:
(a) a MCRA, in a pharmaceutical composition in admixture with a pharmaceutical carrier or vehicle; and
(b) a naAEA consisting of a 5HT3-antagonist selected from the group consisting of alosetron and pharmaceutically acceptable salts and solvates thereof, in particular the hydrochloride, in an amount (in alosetron) of from 0.25 mg to 6 mg; azasetron and pharmaceutically acceptable salts and solvates thereof, in particular the hydrochloride, in an amount (in azasetron) of from 5 mg to 60 mg; dolasetron and pharmaceutically acceptable salts and solvates thereof, in particular the mesylate, in an amount (in dolasetron) of from 25 mg to 600 mg; granisetron and pharmaceutically acceptable salts and solvates thereof, in particular the hydrochloride, in an amount (in granisetron) of from 0.5 mg to 6 mg; ondansetron and pharmaceutically acceptable salts and solvates thereof, in particular the hydrochloride dihydrate, in an amount (in ondansetron) of from 2 mg to 64 mg; palonosetron and pharmaceutically acceptable salts thereof, in particular the hydrochloride, in an amount (in palonosetron) of from 0.25 mg to 3 mg; ramosetron and pharmaceutically acceptable salts thereof, in particular its hydrochloride, in an amount (in ramosetron) of from 0.0125 mg to 0.3 mg, tropisetron and pharmaceutically acceptable salts and solvates thereof, in particular the hydrochloride, in an amount of from 2.5 mg to 30 mg, in a pharmaceutical composition in admixture with a pharmaceutical carrier or vehicle.

A specific MCRA/naAEA combination comprises
(a) a MCRA, in a pharmaceutical composition in admixture with a pharmaceutical carrier or vehicle;
(b) a naAEA consisting of a fixed-dose combination comprising palonosetron, in an amount of from 0.25 mg to 3 mg of palonosetron or a pharmaceutically acceptable salt thereof such as its hydrochloride and from 150 mg to 600 mg of netupitant, in admixture with a pharmaceutical carrier in an oral IR formulation.

According to this second embodiment, another advantageous combination is a combination comprising or consisting essentially of:
(a) a MCRA, in a pharmaceutical composition in admixture with a pharmaceutical carrier or vehicle; and
(b) a naAEA consisting of a DA-antagonist consisting of domperidone and pharmaceutically acceptable salts and solvates thereof such as the maleate; metoclopramide and pharmaceutically acceptable salts and solvates thereof such as the hydrochloride monohydrate, bromopride and pharmaceutically acceptable salts and solvates thereof such as the monohydrochloride or the dihydrochloride monohydrate, alizapride and pharmaceutically acceptable salts and solvates thereof such as the hydrochloride, and clebopride and pharmaceutically acceptable salts and solvates thereof such as the malate and the hydrochloride monohydrate; in a pharmaceutical composition in admixture with a pharmaceutical carrier.

An advantageous combination according to this second embodiment is a combination comprising or consisting essentially of the following Components:
(a) a MCRA, in a pharmaceutical composition in admixture with a pharmaceutical carrier or vehicle; and
(b) a naAEA consisting of a DA-antagonist consisting of domperidone or a pharmaceutically acceptable salt thereof, in particular its maleate, in an amount (in domperidone) of from 5 mg to 20 mg per dosage unit in an IR unit form or in an amount of from 7.5 mg to 60 mg, preferably from 10 mg to 60 mg, in an ER unit form; metoclopramide or a pharmaceutically acceptable salt or solvate thereof, in particular its monohydrochloride monohydrate, in an amount (in metoclopramide) of from 5 mg to 20 mg per dosage unit in an IR unit form or in an amount of from 7.5 mg to 30 mg, preferably from 10 mg to 30 mg, in an ER unit form; alizapride or a pharmaceutically acceptable salt thereof, in particular its hydrochloride, in an amount (in alizapride) of from 25 mg to 100 mg per dosage unit in an IR unit form or in an amount of from 37.5 mg to 300 mg, preferably from 100 mg to 300 mg, in an ER unit form; in a pharmaceutical composition in admixture with a pharmaceutical carrier.

A further advantageous combination according to this second embodiment, is a combination comprising or consisting essentially of the following Components:
(a) a MCRA, in a pharmaceutical composition in admixture with a pharmaceutical carrier or vehicle;
(b) a naAEA consisting of a histamine H1 receptor antagonists selected from the group consisting of meclizine (meclozine) and pharmaceutically acceptable salts and solvates thereof such as the hydrochloride monohydrate; promethazine and pharmaceutically acceptable salts and solvates thereof such as the hydrochloride; chlorpromazine and pharmaceutically acceptable salts and solvates thereof such as the hydrochloride, prochlorperazine and pharmaceutically acceptable salts and solvates thereof such as the dimaleate, the dimesylate or the 1,2-ethanedisulfonate (1:1) (edisilate); hydroxyzine and pharmaceutically acceptable salts and solvates thereof such as the dihydrochloride or the 1,1'-methylene bis(2-hydroxy-3-naphthalenecarboxylic acid salt (pamoate); in a pharmaceutical composition in admixture with a pharmaceutical carrier.

According to this second embodiment, a further advantageous combination is a combination comprising or consisting essentially of the following Components:
(a) a MCRA, in a pharmaceutical composition in admixture with a pharmaceutical carrier or vehicle; and
(b) a naAEA consisting of a histamine H1 receptor antagonists selected from the group consisting of meclizine or a pharmaceutically acceptable salt thereof, in particular its hydrochloride, in an amount (in meclizine) of from 25 mg to 100 mg per dosage unit in an IR unit form or in an amount of from 37.5 mg to 150 mg, preferably from 50 mg to 150 mg, in an ER unit form; chlorpromazine or a pharmaceutically acceptable salt thereof, in particular its hydrochloride, in an amount (in chlorpromazine) of from 50 mg to 200 mg per dosage unit in an IR unit form or in an amount of from 75 mg to 300 mg, preferably from 100 mg to 300 mg, in an ER unit form; prochlorperazine or a pharmaceutically acceptable salt thereof, in particular its maleate, in an amount (in prochlorperazine) of from 2.5 mg to 10 mg per dosage unit in an IR unit form or in an amount of from 3.75 mg to 15 mg, preferably from 5 mg to 15 mg, in an ER unit form; in a pharmaceutical composition in admixture with a pharmaceutical carrier.

A preferred combination according to this second embodiment is a combination comprising or consisting essentially of the following Components:
(a) a MCRA, in a pharmaceutical composition in admixture with a pharmaceutical carrier or vehicle; and
(b) a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount(in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

The pharmaceutical combination according to this second embodiment are indicated in the treatment of hypocholinergic disorders and even high doses of a MCRA Component (a), may be present to improve said symptoms without adverse effects to a greater extent.

Thus, the present invention provides a method for treating hypocholinergic disorders, which comprises administering to a patient in need of said treatment the combinations described according to this second embodiment. In such a treatment, Component (a) and Component (b) of the combination may be administered simultaneously or sequentially to said patient, Component (a) being indifferently administered before or after Component (b). Component (a) and Component (b) may also be administered by the same or a different administration route.

According to a third embodiment, an advantageous combination may be a combination comprising or consisting essentially of
(a) a pharmaceutical composition comprising cevimeline, as free base or as its hydrochloride hemihydrate, in an amount of from 36 mg to 180 mg, in an IR-formulated oral composition in admixture with a pharmaceutical carrier; and
(b) a pharmaceutical composition comprising a naAEA selected from the group consisting of alosetron and pharmaceutically acceptable salts and solvates thereof, azasetron and pharmaceutically acceptable salts and solvates thereof; ondansetron and pharmaceutically acceptable salts and solvates thereof; granisetron and pharmaceutically acceptable salts and solvates thereof; dolasetron and pharmaceutically acceptable salts and solvates thereof, ramosetron and pharmaceutically acceptable salts and solvates thereof; tropisetron and pharmaceutically acceptable salts and solvates thereof; and palonosetron and pharmaceutically acceptable salts and solvates thereof, domperidone and pharmaceutically acceptable salts and solvates thereof such as the maleate; chlorpromazine and pharmaceutically acceptable salts and solvates thereof such as the hydrochloride; prochlorperazine and its salts and solvates, particularly the dimaleate and the dimesylate; promethazine and pharmaceutically acceptable salts and solvates thereof such as the hydrochloride; metoclopramide and pharmaceutically acceptable salts and solvates thereof such as the hydrochloride monohydrate, bromopride and pharmaceutically acceptable salts and solvates thereof such as the monohydrochloride or the dihydrochloride monohydrate, alizapride and pharmaceutically acceptable salts and solvates thereof such as the hydrochloride, and clebopride and pharmaceutically acceptable salts and solvates thereof such as the malate and the hydrochloride monohydrate; dronabinol; nabilone; aprepitant; netupitant; rolapitant; and casopitant, in admixture with a pharmaceutical carrier.

An advantageous combination according to this third embodiment may be a combination comprising or consisting essentially of, as Components:
(a) a pharmaceutical composition comprising cevimeline, as free base or as its hydrochloride hemihydrate, in an amount of from 36 mg to 180 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount(in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Another advantageous combination according to this third embodiment may be a combination comprising or consisting essentially of, as Components:
(a) a pharmaceutical composition comprising cevimeline, as free base or as its hydrochloride hemihydrate, in an amount of from 36 mg to 180 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Preferably, according to this third embodiment, the pharmaceutical composition Component (a) comprises cevimeline, hydrochloride hemihydrate, in an amount of from 36 mg to 180 mg, in an IR-formulated oral composition in admixture with a pharmaceutical carrier and Component (b) comprises a fixed-dose combination consisting of palonosetron hydrochloride, in an amount of 0.5 mg, and netupitant, in an amount of 300 mg.

According to a fourth embodiment, an advantageous MCRA/naAEA combination according to the present invention may be a combination comprising or consisting essentially of
(a) a pharmaceutical composition comprising xanomeline, as free base, as oxalate or as L-tartrate, in an amount of from 90 mg to 450 mg, normally from 90 mg to 300 mg, in admixture with a pharmaceutical carrier; and
(b) a pharmaceutical composition comprising a naAEA selected from the group consisting of alosetron and pharmaceutically acceptable salts and solvates thereof; azasetron and pharmaceutically acceptable salts and solvates thereof; ondansetron and pharmaceutically acceptable salts and solvates thereof; granisetron and pharmaceutically acceptable salts and solvates thereof; dolasetron and pharmaceutically acceptable salts and solvates thereof; ramosetron and pharmaceutically acceptable salts and solvates thereof; tropisetron and pharmaceutically acceptable salts and solvates thereof; palonosetron and pharmaceutically acceptable salts and solvates thereof; domperidone and pharmaceutically acceptable salts and solvates thereof such as the maleate; chlorpromazine and pharmaceutically acceptable salts and solvates thereof such as the hydrochloride; prochlorperazine and its salts and solvates, particularly the dimaleate and the dimesylate; promethazine and pharmaceutically acceptable salts and solvates thereof such as the hydrochloride; metoclopramide and pharmaceutically acceptable salts and solvates thereof such as the hydrochloride monohydrate; bromopride and pharmaceutically acceptable salts and solvates thereof such as the monohydrochloride or the dihydrochloride monohydrate; alizapride and pharmaceutically acceptable salts and solvates thereof such as the hydrochloride; clebopride and pharmaceutically acceptable salts and solvates thereof such as the malate and the hydrochloride monohydrate; dronabinol; nabilone; aprepitant; netupitant; rolapitant; and casopitant
in admixture with a pharmaceutical carrier.

An advantageous combination according to this fourth embodiment may be a combination comprising or consisting essentially of, as Components:
(a) a pharmaceutical composition comprising xanomeline, as free base, as oxalate or as L-tartrate, in an amount of from 90 mg to 450 mg, normally from 90 mg to 300 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg; tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Another advantageous combination according to this fourth embodiment may be a combination comprising or consisting essentially of, as Components:
(a) a pharmaceutical composition comprising xanomeline, as free base, as oxalate or as L- hydrogen tartrate, in an amount of from 90 mg to 450 mg, normally from 90 mg to 300 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg; tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Preferably, according to this fourth embodiment, the pharmaceutical composition Component (a) comprises xanomeline, as free base, as oxalate or as L-tartrate, in an amount of from 90 mg to 450 mg, normally from 90 mg to 300 mg, in admixture with a pharmaceutical carrier in an IR or ER oral formulation and the pharmaceutical composition Component (b) comprises a fixed-dose combination consisting of palonosetron hydrochloride, in an amount of 0.5 mg, and netupitant, in an amount of 300 mg in admixture with a pharmaceutical carrier in an oral formulation. A particular pharmaceutical composition according to this fourth embodiment comprises xanomeline base in admixture with a pharmaceutical carrier or vehicle for ER administration, said composition being in a patch delivering a predetermined xanomeline dose over 24 hours. An advantageous predetermined xanomeline dose is released in an amount/24h giving xanomeline plasma concentrations in human of from 16.572ng/ml to 78.6ng/ml.

According to a fifth embodiment, an advantageous MCRA/naAEA combination may be a combination comprising or consisting essentially of
(a) milameline hydrochloride, in an amount of from 2.4 mg to 12 mg, normally from 2.4 to 10 mg, in admixture with a pharmaceutical carrier; and
(b) a pharmaceutical composition comprising a naAEA selected from the group consisting of alosetron and pharmaceutically acceptable salts and solvates thereof; azasetron and pharmaceutically acceptable salts and solvates thereof; ondansetron and pharmaceutically acceptable salts and solvates thereof; granisetron and pharmaceutically acceptable salts and solvates thereof; dolasetron and pharmaceutically acceptable salts and solvates thereof; ramosetron and pharmaceutically acceptable salts and solvates thereof; tropisetron and pharmaceutically acceptable salts and solvates thereof; and palonosetron and pharmaceutically acceptable salts and solvates thereof; domperidone and pharmaceutically acceptable salts and solvates thereof such as the maleate; chlorpromazine and pharmaceutically acceptable salts and solvates thereof such as the hydrochloride; prochlorperazine and its salts and solvates, particularly the dimaleate and the dimesylate; promethazine and pharmaceutically acceptable salts and solvates thereof such as the hydrochloride; metoclopramide and pharmaceutically acceptable salts and solvates thereof such as the hydrochloride monohydrate; bromopride and pharmaceutically acceptable salts and solvates thereof such as the monohydrochloride or the dihydrochloride monohydrate; alizapride and pharmaceutically acceptable salts and solvates thereof such as the hydrochloride; clebopride and pharmaceutically acceptable salts and solvates thereof such as the malate and the hydrochloride monohydrate; dronabinol; nabilone; aprepitant; netupitant; rolapitant; and casopitant.

According to a further aspect of this fifth embodiment, an advantageous MCRA/naAEA combination may be a combination comprising or consisting essentially of
(a) a pharmaceutical composition comprising milameline hydrochloride, in an amount of from 2.4 mg to 12 mg, normally from 2.4 to 10 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg; tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Preferably, according to this fifth embodiment, the pharmaceutical composition Component (a) comprises a pharmaceutical composition comprising milameline, as free base or as hydrochloride, in an amount of from 2.4 mg to 12 mg, normally from 2.4 to 10 mg, in admixture with a pharmaceutical carrier in an IR or ER oral formulation; and the pharmaceutical composition Component (b) comprises a fixed-dose combination consisting of palonosetron hydrochloride, in an amount of 0.5 mg, and netupitant, in an amount of 300 mg in admixture with a pharmaceutical carrier in an oral formulation.

According to a sixth embodiment, an advantageous MCRA/naAEA combination according to the present invention is a combination comprising or consisting essentially of
(a) MK-7622, as free base, as hydrochloride, as monomethanesulfonate or as fumarate, in an amount consisting of from 5 mg to 270 mg, in particular of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in an IR-formulated oral composition in admixture with a pharmaceutical carrier; and
(b) a pharmaceutical composition comprising a naAEA selected from the group consisting of alosetron and pharmaceutically acceptable salts and solvates thereof, azasetron and pharmaceutically acceptable salts and solvates thereof; ondansetron and pharmaceutically acceptable salts and solvates thereof; granisetron and pharmaceutically acceptable salts and solvates thereof; dolasetron and pharmaceutically acceptable salts and solvates thereof, ramosetron and pharmaceutically acceptable salts and solvates thereof; tropisetron and pharmaceutically acceptable salts and solvates thereof; and palonosetron and pharmaceutically acceptable salts and solvates thereof, domperidone and pharmaceutically acceptable salts and solvates thereof such as the maleate; chlorpromazine and pharmaceutically acceptable salts and solvates thereof such as the hydrochloride; prochlorperazine and its salts and solvates, particularly the dimaleate and the dimesylate; promethazine and pharmaceutically acceptable salts and solvates thereof such as the hydrochloride; metoclopramide and pharmaceutically acceptable salts and solvates thereof such as the hydrochloride monohydrate, bromopride and pharmaceutically acceptable salts and solvates thereof such as the monohydrochloride or the dihydrochloride monohydrate, alizapride and pharmaceutically acceptable salts and solvates thereof such as the hydrochloride, and clebopride and pharmaceutically acceptable salts and solvates thereof such as the malate and the hydrochloride monohydrate, dronabinol, nabilone, aprepitant, netupitant, rolapitant, and casopitant, in admixture with a pharmaceutical carrier.

According to a further aspect of this sixth embodiment, an advantageous MCRA/naAEA combination may be a combination comprising or consisting essentially of
(a) a pharmaceutical composition comprising MK-7622, as free base, as hydrochloride, as monomethanesulfonate or as fumarate, in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg; tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier.

Another advantageous MCRA/naAEA combination may be a combination comprising or consisting essentially of
(a) a pharmaceutical composition comprising MK-7622, as free base, as hydrochloride or as fumarate, in an amount of from 5 mg to 270 mg, in particular in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in admixture with a pharmaceutical carrier; and
(b) a pharmaceutical composition comprising a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg; tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in admixture with a pharmaceutical carrier.

Advantageously, according to this sixth embodiment, the pharmaceutical composition comprises the MK-7622 Component (a) in an amount of from 5 mg to 270 mg, in particular in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in admixture with a pharmaceutical carrier in an oral IR or ER formulation and the naAEA Component (b) is a fixed-dose combination consisting of palonosetron hydrochloride, in an amount of 0.5 mg, and netupitant, in an amount of 300 mg, in admixture with a pharmaceutical carrier in an oral formulation. Preferably, Component (a) comprises MK-7622, as free base, as hydrochloride or as fumarate, in an amount from 6 mg to 270 mg, normally from more than 45 mg to 180 mg, in admixture with a pharmaceutical carrier in an oral IR or ER formulation.

According to a seventh embodiment, an advantageous MCRA/naAEA combination is a fixed-dose combination essentially consisting of a pharmaceutical composition in dosage unit form comprising
- a MCRA Component (a); and
- a naAEA Component (b),
in admixture with a pharmaceutical carrier or vehicle.

In the fixed-dose combination, the MCRA Component (a) and the naAEA Component (b) may be any one of the corresponding compounds illustrated in the above "The MCRA Component (a)" and "The naAEA Component (b)".

An advantageous MCRA/naAEA composition in dosage unit form comprises or essentially consists of
(i) a MCRA selected from the group consisting of AF267 and pharmaceutically acceptable salts and solvates thereof; cevimeline and pharmaceutically acceptable salts and solvates thereof; 3-[3-(3-(3-fluorophenyl)-2-propyn-1-ylthio)-1,2,5-thiadiazol-4-yl]-1,2,5,6-tetrahydro-1-methylpyridine and pharmaceutically acceptable salts and solvates thereof; milameline and pharmaceutically acceptable salts and solvates thereof; RS-86 and pharmaceutically acceptable salts and solvates thereof; sabcomeline and pharmaceutically acceptable salts and solvates thereof; talsaclidine and pharmaceutically acceptable salts and solvates thereof; 5-[4-(hexylsulfanyl)-1,2,5-thiadiazol-3-yl]-1-methyl-1,2,3,6-tetrahydropyridine and pharmaceutically acceptable salts and solvates thereof; xanomeline and pharmaceutically acceptable salts and solvates thereof; MCD-386 and pharmaceutically acceptable salts and solvates thereof; 3-Methyl-5-(piperidin-3-yl)-1,2,4-oxadiazole, its enantiomers and pharmaceutically acceptable salts and solvates thereof; MK-7622 and pharmaceutically acceptable salts and solvates thereof; and
(ii) a naAEA selected from the group consisting of (b1) 5HT3-antagonists, (b2) DA-antagonists, (b3) H1-antagonists, (b4) cannabinoids, (b5) NK1-antagonists,
in admixture with a pharmaceutical carrier.

A particularly advantageous MCRA/naAEA composition in dosage unit form comprises or essentially consists of
(i) a MCRA selected from the group consisting of cevimeline hydrochloride hemihydrate, in an amount of from 34.5 mg to 180 mg, xanomeline, as free base, as oxalate or as L-tartrate, in an amount of from 90 mg to 450 mg, milameline hydrochloride, in an amount of from 2.4 mg to 12 mg and MK 7622, as free base, as hydrochloride, as fumarate or as methanesulfonate, in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg; and
(ii) a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg,
in admixture with a pharmaceutical carrier.

A particularly advantageous MCRA/naAEA fixed-dose combination according to this aspect of this seventh embodiment consists of a transdermal patch comprising xanomeline and granisetron.

Any of the above MCRA/naAEA combinations may contain, as a further component, Component (c), an AChEI also formulated in a pharmaceutical composition, said AChEI may include, but is not limited to, 1,2,3,4-tetrahydro-9-acridinamine (tacrine) and pharmaceutically acceptable salts and solvates thereof, (±)-2,3-dihydro-5,6-dimethoxy-2-[[1-(phenylmethyl)-4-piperidinyl]methyl]-1H-inden-1-one (donepezil) and pharmaceutically acceptable salt and solvates thereof, (*S*)-N-Ethyl-N-methyl-3-[1-(dimethylamino)ethyl]-phenyl carbamate (rivastigmine) and pharmaceutically acceptable salts and solvates thereof, or 4aS,6R,8aS-3-methoxy-11-methyl-4a,5,9,10,11,12-hexahydroxy-6H-benzofuro[3a,3,2-e,f]benzazepin-6-ol (galantamine) and pharmaceutically acceptable salts and solvates thereof.

The AChEI Component (c) when included with Component (a) and Component (b) as described herein, may be present in an amount of from about 100% to about 1000% of a recommended dose of said AChEI Component (c) contained in a unit form used for the treatment of Alzheimer type dementia.

Among the particularly preferred AChEIs, in the combinations of the present invention donepezil hydrochloride is present at a dose of from 10 mg to 98 mg, advantageously from 15 mg to 69 mg, normally from 15 mg to 60 mg; rivastigmine, as hydrogen tartrate, is present, in a composition for oral administration, at a dose of from 6 mg to 30 mg, advantageously from 9 mg to 24 mg, normally 9 mg to 18 mg; as the free base, rivastigmine is present in an amount of from 9 mg to 54 mg, in patch releasing from 4.6mg/24h to 52mg/24h rivastigmine, advantageously from 9.6mg/24h to 39.9mg/24h, normally from 13.3mg/24h to 39.9mg/24h.

In said combination, said AChEI Component (c) may be formulated, in admixture with a pharmaceutical carrier or vehicle, in a pharmaceutical composition or device in dosage unit form or also used as a brand preparation.

For example, rivastigmine may be also used by orally administering EXELON^{®} immediate-release 6 mg-capsules or by applying one or more EXELON^{®} patches releasing 4.6 mg/24 hours, 9.5 mg/24 hours, or 13.3 mg/24 hours on the subject's skin, to daily release rivastigmine at a dose/24h of from 4.6 mg to 53.2 mg or from 19.95 mg to 53.2 mg, normally from 14.1 mg to 46 mg, in combination with the above-illustrated MCRA/naAEA combination.

Donepezil hydrochloride may be also used by orally administering one or more ARICEPT^{®} immediate-release 5 mg- or 10 mg-tablets or the 23-mg tablets. In particular, donepezil hydrochloride may be orally administered, in combination with the above-illustrated MCRA/naAEA combination, at a daily dose of from 5 mg to 100 mg or from 15 mg to 70 mg.

Similarly, galantamine (as hydrobromide) may be also administered as a brand preparation, for example by orally administering RAZADYNE^{®} immediate-release 8 mg- or 12 mg-tablets or RAZADYNE^{®} ER 8 mg-, 16 mg- or 24 mg-capsules. In particular, galantamine hydrobromide may be orally administered, in combination with the above-illustrated MCRA/naAEA combination, at a daily dose (in galantamine) of from 36 mg to 96 mg, normally at a daily dose or from 36 mg to 72 mg, preferably in an ER-form.

The present invention also provides a fixed-dose combination that is a pharmaceutical composition in dosage unit form comprising or consisting essentially of, as Components:
(a) a muscarinic cholinergic receptor agonist (MCRA);
(b) a non-anticholinergic antiemetic agent (naAEA); and
(c) an AChEI,
in admixture with at least one pharmaceutical carrier.

This pharmaceutical composition of the present invention improves the treatment of human hypocholinergic disorders of the CNS as described above, such as dementias of the Alzheimer type and schizophrenia. Any MCRA, any naAEA and any AChEI as described herein, and exemplified in the above "The Combinations" section may be formulated in a pharmaceutical composition in a single unit form, in admixture with at least one pharmaceutical carrier according to conventional methods in the art, and as exemplified in the "The Formulations" section below.

In a preferred embodiment, Components (a) are the aforementioned MCRAs, in particular AF267 and pharmaceutically acceptable salts and solvates thereof, especially its hydrochloride (AF 267B), cevimeline and pharmaceutically acceptable salts and solvates thereof, 3-[3-(3-(3-fluorophenyl)-2-propyn-1-ylthio)-1,2,5-thiadiazol-4-yl]-1,2,5,6-tetrahydro-1-methylpyridine (EUK 1001) and pharmaceutically acceptable salts and solvates thereof especially its oxalate, milameline and pharmaceutically acceptable salts and solvates thereof especially its hydrochloride, RS-86 and pharmaceutically acceptable salts and solvates thereof, especially its hydrobromide; sabcomeline and pharmaceutically acceptable salts and solvates thereof; talsaclidine and pharmaceutically acceptable salts and solvates thereof, especially its fumarate; tazomeline and pharmaceutically acceptable salts and solvates thereof, especially its hydrochloride; xanomeline and pharmaceutically acceptable salts and solvates thereof, especially its oxalate and its L-tartrate; racemic 3-Methyl-5-(piperidin-3-yl)-1,2,4-oxadiazole and pharmaceutically acceptable salts and solvates thereof, S-(+)-3-methyl-5-(piperidin-3-yl)-1,2,4-oxadiazole D-tartrate; R-(-)-3-Methyl-5-(piperidin-3-yl)-1,2,4-oxadiazole L-tartrate; and MK-7622 and pharmaceutically acceptable salts and solvates thereof, especially its hydrochloride and its fumarate.

In a particularly preferred embodiment, Component (a) is a MCRA selected from the group consisting of cevimeline and pharmaceutically acceptable salts thereof, milameline and pharmaceutically acceptable salts and solvates thereof, xanomeline and pharmaceutically acceptable salt and solvates thereof; racemic 3-Methyl-5-(piperidin-3-yl)-1,2,4-oxadiazole and pharmaceutically acceptable salts and solvates thereof, S-(+)-3-methyl-5-(piperidin-3-yl)-1,2,4-oxadiazole D-tartrate; R-(-)-3-Methyl-5-(piperidin-3-yl)-1,2,4-oxadiazole L-tartrate; MK-7622 and pharmaceutically acceptable salts and solvates thereof. Cevimeline, its hydrochloride hemihydrate, xanomeline, its oxalate or L-tartrate and MK-7622, its hydrochloride or fumarate or methanesulfonate are the preferred Components (a) of the fixed-dose combination. The Component (b) of the fixed-dose combination may be a non-anticholinergic antiemetic agent selected from the group consisting of (b1) 5HT3-antagonists, (b2) DA-antagonists, (b3) H1-antagonists, (b4) cannabinoids, (b5) NK1-antagonists.

In a preferred embodiment, Component (b) of the fixed-dose combination is selected from the group consisting of alosetron and pharmaceutically acceptable salts and solvates thereof, especially its hydrochloride; azasetron and pharmaceutically acceptable salts and solvates thereof, especially its hydrochloride; ondansetron and pharmaceutically acceptable salts and solvates thereof, especially its monohydrochloride dihydrate; granisetron and pharmaceutically acceptable salts and solvates thereof, especially its hydrochloride; dolasetron and pharmaceutically acceptable salts and solvates thereof, especially its monomethanesulfonate monohydrate, ramosetron and pharmaceutically acceptable salts and solvates thereof, especially its hydrochloride; tropisetron and pharmaceutically acceptable salts and solvates thereof, especially its hydrochloride; palonosetron and pharmaceutically acceptable salts and solvates thereof, especially its hydrochloride; domperidone and pharmaceutically acceptable salts and solvates thereof, especially its maleate; metoclopramide and pharmaceutically acceptable salts and solvates thereof, especially its hydrochloride monohydrate; bromopride and pharmaceutically acceptable salts and solvates thereof, especially its monohydrochloride or the dihydrochloride monohydrate; alizapride and pharmaceutically acceptable salts and solvates thereof, especially its hydrochloride; clebopride and pharmaceutically acceptable salts and solvates thereof, especially its malate and the hydrochloride monohydrate; meclizine (meclozine) and pharmaceutically acceptable salts and solvates thereof, especially its hydrochloride monohydrate; promethazine and pharmaceutically acceptable salts and solvates thereof, especially its hydrochloride; chlorpromazine and pharmaceutically acceptable salts and solvates thereof, especially its hydrochloride; prochlorperazine and pharmaceutically acceptable salts and solvates thereof, especially its dimaleate, its dimesylate and its the 1,2-ethanedisulfonate (1:1) (edisilate); hydroxyzine and pharmaceutically acceptable salts and solvates thereof such as the dihydrochloride or the 4-[(3-carboxy-2-hydroxynaphthalen-1-yl)methyl]-3-hydroxynaphthalene-2-carboxylate (pamoate) dronabinol; nabilone; aprepitant; netupitant; rolapitant; and casopitant.

In another preferred embodiment, Component (c) of the fixed-dose combination is an AChEI selected form the group consisting of donepezil and pharmaceutically acceptable salts thereof, rivastigmine and pharmaceutically acceptable salts thereof, and galantamine and pharmaceutically acceptable salts thereof.

Among the preferred Components (a) of the fixed-dose combination,
- cevimeline is present, as hydrochloride hemihydrate, in an amount of from 30 mg to 120 mg, preferably from 36 mg to 120 mg in an oral IR or, as free base or as hydrochloride hemihydrate, from 36 mg to 180 mg, preferably from 45 mg to 180 mg in an oral or transdermal ER form, in particular in a TTS;
- milameline is present, as hydrochloride, in an amount of from 2 mg to 8 mg, preferably from 2.4 mg to 8 mg in an oral IR or, as free base or as hydrochloride, from 2.4 mg to 12 mg, preferably from 3 mg to 12 mg in an oral or transdermal ER form, in particular in a TTS;
- xanomeline is present, as oxalate or L-tartrate, in an amount of from 75 mg to 300 mg preferably from 90 mg to 300 mg in an oral IR form or, as free base, as oxalate or as L-tartrate, from 90 mg to 450 mg, preferably from 112.5 mg to 450 mg in an oral or transdermal ER form, in particular in a TTS; and
- MK-7622, as free base, as hydrochloride or as fumarate, in an amount of from 5 mg to 270 mg, in particular in an amount selected from the group consisting of more than 5 mg to 15 mg, from more than 15 mg to 45 mg, from more than 45 mg to 270 mg, from more than 15 mg to 225 mg, from more than 45 mg to 225 mg, and from 54 mg to 180 mg.

Preferably, Component (b) of the fixed-dose combination is selected from the group consisting of ondansetron and pharmaceutically acceptable salts and solvates thereof, in an amount (in ondansetron) of from 8 mg to 64 mg; granisetron and pharmaceutically acceptable salts and solvates thereof, in an amount (in granisetron) of from 1 mg to 3 mg; domperidone and pharmaceutically acceptable salts and solvates thereof, in an amount (in domperidone) of from 10 mg to 30 mg; metoclopramide and pharmaceutically acceptable salts and solvates thereof, in an amount (in metoclopramide) of from 10 mg to 30 mg; dronabinol, in an amount of from 10 mg to 30 mg; nabilone, in an amount of from 1 mg to 3 mg; aprepitant, in an amount of from 125 mg to 375 mg; netupitant, in an amount of from 200 mg to 600 mg; rolapitant, in an amount of form 60 mg to 180 mg; and casopitant, in an amount of from 50 mg to 150 mg.

An AChEI selected from the group consisting of donepezil hydrochloride, in an amount of from 5 mg to 40 mg, rivastigmine, as free base or as hydrogen tartrate, in an amount (in rivastigmine) of from 1.5 mg to 52 mg; and galantamine hydrobromide, in an amount (in galantamine) of from 4 mg to 48 mg; is the preferred Component (c) in the fixed-dose combination.

The above fixed-dose combination and any of the pharmaceutical compositions that are part of the above combinations are formulated with pharmaceutical carriers, diluents, vehicles and devices according to known and conventional methods and/or technologies in the art and as illustrated in the "The Formulations" section below.

### The Combinations in Kits

The present invention also provides a kit or package containing a combination as described herein, accompanied by instructions for use. In particular, a kit of the present invention is a kit comprising a combination of medicaments for the treatment of hypocholinergic disorders of the CNS.

According to the present invention, the kit allows for the maximal functional capacity and safety during the treatment of a patient with a combination wherein the components may be administered concurrently or sequentially.

More particularly, the kit of the present invention comprises
(a) a pharmaceutical composition in IR or ER dosage unit form comprising or consisting essentially of a therapeutically effective amount of a MCRA in admixture with a pharmaceutical carrier; and
(b) a pharmaceutical composition in IR or ER dosage unit form comprising or consisting essentially of a therapeutically effective amount of a naAEA in admixture with a pharmaceutical carrier;
for concurrent, sequential or separate administration.

The pharmaceutical compositions may be packaged in any manner suitable for administration to a patient suffering from a hypocholinergic disorder of the CNS dementia and the packaging is manufactured according to known technologies and completed with instructions for use clearly showing to the patient or to the caregiver how to take each of the units forms to be administered.

Said kit comprises a Component (a) selected among the MCRAs illustrated in the above section "The MCRAs" and a Component (b) selected among the naAEAs illustrated in the above section "The naAEAs".

Component (a) and Component (b) may be present in the kit both in IR or in ER form or one of the Components is in IR form and the other is in ER form, each in admixture with a pharmaceutical carrier in a composition formulated as illustrated in "The Formulations" section, according to known technologies.

The kit according to the present invention may also comprise an AChEI Component (c), also in an IR or ER form, in admixture with a pharmaceutical carrier in a composition formulated as illustrated in "The Formulations" section below, according to known technologies.

According to a first embodiment, the kit of the present invention comprises
(a) a MCRA selected from the group consisting of AF267 and pharmaceutically acceptable salts and solvates thereof; cevimeline and pharmaceutically acceptable salts and solvates thereof; EUK 1001 and pharmaceutically acceptable salts and solvates thereof; milameline and pharmaceutically acceptable salts and solvates thereof; RS-86 and pharmaceutically acceptable salts and solvates thereof; sabcomeline and pharmaceutically acceptable salts and solvates thereof; talsaclidine and pharmaceutically acceptable salts and solvates thereof; tazomeline and pharmaceutically acceptable salts and solvates thereof; xanomeline and pharmaceutically acceptable salts and solvates thereof; AC-42 and pharmaceutically acceptable salts and solvates thereof; TBPB and pharmaceutically acceptable salts and solvates thereof; 4-Fluoro-6-methyl-1-[1-(tetrahydro-2H-pyran-4-yl)-4-piperidinyl]-1,3-dihydro-2H- benzimidazol-2-one and pharmaceutically acceptable salts and solvates thereof; 5-Fluoro-6-methyl-1-[1-(tetrahydro-2H-pyran-4-yl)-4-piperidinyl]-1,3-dihydro-2H-benzimidazol-2-one and pharmaceutically acceptable salts and solvates thereof; 4-(R)-ethyl-3-(2-methylbenzamido)-1,4'-bipiperidine-1'-carboxylate and pharmaceutically acceptable salts and solvates thereof; ethyl 3-[(3-exo)-(2-benzamidoethyl)amino]-8-azabicyclo[3.2.1]octane-8-carboxylate and pharmaceutically acceptable salts and solvates thereof; and MK-7622; and
(b) a naAEA selected from the group consisting of a naAEA selected from the group consisting of (b1) 5HT3-antagonists, (b2) DA-antagonists, (b3) H1-antagonists, (b4) cannabinoids, (b5) NK1-antagonists and; optionally,
(c) an AChEI selected from the group consisting of donepezil and its pharmaceutically acceptable salts, rivastigmine and its pharmaceutically
acceptable salts, and galantamine and its pharmaceutically acceptable salts; Component (a) and Component (b) being in a fixed-dose combination formulated in admixture with a pharmaceutical carrier.

When the AChEI Component (c) is present in the kit, it is in a separate unit form wherein said AChEI is mixed with a pharmaceutical carrier in a pharmaceutical composition formulated in an IR or ER unit form.

According to this first embodiment, the combination is administered to a patient in need of the treatment as a single unit form for each of the Component (a), Component (b) and Component (c), at the doses illustrated in "The Combinations" section.

According to a second embodiment, the invention provides a kit comprising
(a) a pharmaceutical composition in dosage unit form comprising or consisting essentially of a MCRA in admixture with a pharmaceutical carrier; and
(b/c) a fixed-dose combination that is a pharmaceutical composition comprising or consisting essentially of
   (b) a naAEA; and
   (c) an AChEI,
   in admixture with a pharmaceutical carrier.

This kit has the great advantage of allowing an improvement in the treatment of a patient suffering from a hypocholinergic disorder. In fact, in the case of the prescription of a MCRA that must be taken three or four times/day the kit of the present invention allows the administration of a composition (b/c) comprising a naAEA and an AChEI that may be administered once or twice a day, thus rendering the treatment easier for the patient or for the caregiver.

For example, a kit of the present invention may comprise:
(a) a pharmaceutical composition in dosage unit form comprising or consisting essentially of a MCRA selected from the group consisting of 1-methylpiperidine-4-spiro-5'(2'-ethyl-1',4'-thiazoline-3'-one) (AF267) and pharmaceutically acceptable salts and solvates thereof; cis-2'-methylspiro {1-azabicyclo[2.2.2]octane-3,5'-[1,3]oxathiolane} (cevimeline) and pharmaceutically acceptable salts and solvates thereof; 3-[3-(3-(3-fluorophenyl)-2-propyn-1-ylthio)-1,2,5-thiadiazol-4-yl]-1,2,5,6-tetrahydro-1-methylpyridine and pharmaceutically acceptable salts and solvates thereof; (*E*)-N-methoxy-1-(1-methyl-1,2,5,6-tetrahydropyridin-3-yl)methanimine (milameline) and pharmaceutically acceptable salts and solvates thereof; 2-ethyl-8-methyl-2,8-diazaspiro[4.5] decane-1,3-dione (RS-86) and pharmaceutically acceptable salts and solvates thereof; (3*R*)-N-methoxyquinuclidine-3-carboximidoyl cyanide (sabcomeline) and pharmaceutically acceptable salts and solvates thereof; (3R)-3-(prop-2-yn-1-yloxy)-1-azabicyclo[2.2.2]octane (talsaclidine) and pharmaceutically acceptable salts and solvates thereof; 5-[4-(hexylsulfanyl)-1,2,5-thiadiazol-3-yl]-1-methyl-1,2,3,6-tetrahydropyridine (tazomeline) and pharmaceutically acceptable salts and solvates thereof; 3-(4-hexyloxy-1,2,5-thiadiazol-3-yl)-1-methyl-5,6-dihydro-2*H*-pyridine (xanomeline), and pharmaceutically acceptable salts and solvates thereof; 5-(3-ethyl-1,2,4-oxadiazol-5-yl)-1,4,5,6-tetrahydropyrimidine (MCD-386) and pharmaceutically acceptable salts and solvates thereof; 3-Methyl-5-(piperidin-3-yl)-1,2,4-oxadiazole, its enantiomers and pharmaceutically acceptable salts and solvates thereof; 3-[(1S,2S)-2-hydroxycyclohexyl]-6-[(6-methylpyridin-3-yl)methyl]benzo[h] quinazolin-4(3H)-one (MK-7622) and pharmaceutically acceptable salts and solvates thereof,
in admixture with a pharmaceutical carrier; and
(b/c) a fixed-dose combination that is a pharmaceutical composition comprising or consisting essentially of
   (b) a naAEA selected from the group consisting of alosetron and pharmaceutically acceptable salts and solvates thereof, especially its hydrochloride; azasetron and pharmaceutically acceptable salts and solvates thereof, especially its hydrochloride; ondansetron and pharmaceutically acceptable salts and solvates thereof, especially its monohydrochloride dihydrate; granisetron and pharmaceutically acceptable salts and solvates thereof, especially its hydrochloride; dolasetron and pharmaceutically acceptable salts and solvates thereof, especially its monomethanesulfonate monohydrate, ramosetron and pharmaceutically acceptable salts and solvates thereof, especially its hydrochloride; tropisetron and pharmaceutically acceptable salts and solvates thereof, especially its hydrochloride; palonosetron and pharmaceutically acceptable salts and solvates thereof, especially its hydrochloride; domperidone and pharmaceutically acceptable salts and solvates thereof, especially its maleate; metoclopramide and pharmaceutically acceptable salts and solvates thereof, especially its hydrochloride monohydrate; bromopride and pharmaceutically acceptable salts and solvates thereof, especially its monohydrochloride or the dihydrochloride monohydrate; alizapride and pharmaceutically acceptable salts and solvates thereof, especially its hydrochloride; clebopride and pharmaceutically acceptable salts and solvates thereof, especially its malate and the hydrochloride monohydrate; meclizine (meclozine) and pharmaceutically acceptable salts and solvates thereof, especially its hydrochloride monohydrate; promethazine and pharmaceutically acceptable salts and solvates thereof, especially its hydrochloride; chlorpromazine and pharmaceutically acceptable salts and solvates thereof, especially its hydrochloride; prochlorperazine and pharmaceutically acceptable salts and solvates thereof, especially its dimaleate, its dimesylate and its the 1,2-ethanedisulfonate (1:1) (edisilate); hydroxyzine and pharmaceutically acceptable salts and solvates thereof such as the dihydrochloride or the 1,1'-methylene bis(2-hydroxy-3-naphthalenecarboxylic acid (pamoate) dronabinol; nabilone; aprepitant, netupitant, rolapitant, and casopitant; and
   (c) an AChEI selected from the group consisting of donepezil and its pharmaceutically acceptable salts, rivastigmine and its pharmaceutically acceptable salts, and galantamine and its pharmaceutically acceptable salts,
   in admixture with a pharmaceutical carrier.

A kit of this second embodiment may comprise:
(a) a pharmaceutical composition in dosage unit form comprising or consisting essentially of a MCRA selected from the group consisting of cevimeline hydrochloride hemihydrate, in an amount of from 34.5 mg to 180 mg, xanomeline, as free base, as oxalate or as L-tartrate, in an amount of from 90 mg to 450 mg, milameline hydrochloride, in an amount of from 2.4 mg to 12 mg and MK 7622, as free base, as hydrochloride, as fumarate or as methanesulfonate, in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg,
in admixture with a pharmaceutical carrier; and
(b/c) a fixed-dose combination that is a pharmaceutical composition comprising or consisting essentially of
   (b) a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg; in a pharmaceutical composition in admixture with a pharmaceutical carrier; and
   (c) an AChEI selected from the group consisting of donepezil hydrochloride, in an amount of from 5 mg to 40 mg; rivastigmine hydrogen tartrate, in an amount of from 1.5 mg to 36 mg; and galantamine hydrobromide, in an amount of from 4 mg to 48 mg,
   in admixture with a pharmaceutical carrier.

According to a particular aspect of this second embodiment, the above Component (b/c) is a pharmaceutical composition in dosage unit form wherein said naAEA and said AChEI are mixed together and with a pharmaceutical carrier in an oral IR- or ER-formulation, according to "The Formulations" section below.

According to another particular aspect of this this second embodiment, the above Component (b/c), is a pharmaceutical composition in dosage unit form wherein said naAEA is granisetron and said AChEI is rivastigmine in patch releasing from 0.5mg/24h to 3mg/24h granisetron and from 4.6mg/24h to 52mg/24h rivastigmine, according to "The formulations" section below.

Preferred pharmaceutical compositions that may be used in a kit of this second embodiment may be compositions comprising or consisting essentially of, as Component (b/c), a naAEA (b) and an AChEI (c) in a pharmaceutical composition in dosage unit form as disclosed in US 2011/0201597, the contents of which are incorporated herein by reference in their entirety.

Another advantageous combination comprises or consists essentially of, for example:
(a) a pharmaceutical composition in dosage unit form comprising or consisting essentially of a MCRA consisting of MK 7622 free base, as MK 7622 hydrochloride, MK 7622 fumarate and MK 7622 methanesulfonate, in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, in admixture with a pharmaceutical carrier; and
(b/c) a fixed-dose combination that is a pharmaceutical composition comprising or consisting essentially of
   (b) a naAEA consisting of ondansetron hydrochloride dihydrate, in an amount (in ondansetron) of from 4 mg to 24 mg, preferably from 8 mg to 24 mg; and
   (c) an AChEI consisting of donepezil hydrochloride, in an amount of from 5 mg to 40 mg,
   in admixture with a pharmaceutical carrier.

According to a third embodiment, the invention provides a kit comprising
(a/c) a fixed-dose combination that is a pharmaceutical composition comprising or consisting essentially of
   (a) a MCRA, and
   (c) an AChEI,
   in admixture with a pharmaceutical carrier; and
(b) a pharmaceutical composition in dosage unit form comprising or consisting essentially of a naAEA in admixture with a pharmaceutical carrier.

An advantageous kit according to this third embodiment provides a kit comprising:
(a/c) a fixed-dose combination that is a pharmaceutical composition comprising or consisting essentially of
   (a) a MCRA selected from the group consisting of 1-methylpiperidine-4-spiro-5'(2'-ethyl-1',4'-thiazoline-3'-one) (AF267) and pharmaceutically acceptable salts and solvates thereof; cis-2'-methylspiro {1-azabicyclo [2.2.2] octane-3,5'-[1,3] oxathiolane} (cevimeline) and pharmaceutically acceptable salts and solvates thereof; 3-[3-(3-(3-fluorophenyl)-2-propyn-1-ylthio)-1,2,5-thiadiazol-4-yl]-1,2,5,6-tetrahydro-1-methylpyridine and pharmaceutically acceptable salts and solvates thereof; (*E*)-N-methoxy-1-(1-methyl-1,2,5,6-tetrahydropyridin-3-yl)methanimine (milameline) and pharmaceutically acceptable salts and solvates thereof; 2-ethyl-8-methyl-2,8-diazaspiro[4.5] decane-1,3-dione (RS-86) and pharmaceutically acceptable salts and solvates thereof; (3*R*)-N-methoxyquinuclidine-3-carboximidoyl cyanide (sabcomeline) and pharmaceutically acceptable salts and solvates thereof; (3*R*)-3-(prop-2-yn-1-yloxy)-1-azabicyclo[2.2.2]octane (talsaclidine) and pharmaceutically acceptable salts and solvates thereof; 5-[4-(hexylsulfanyl)-1,2,5-thiadiazol-3-yl]-1-methyl-1,2,3,6-tetrahydropyridine(tazomeline) and pharmaceutically acceptable salts and solvates thereof; 3-(4-hexyloxy-1,2,5-thiadiazol-3-yl)-1-methyl-5,6-dihydro-2*H*-pyridine (xanomeline), and pharmaceutically acceptable salts and solvates thereof; 5-(3-ethyl-1,2,4-oxadiazol-5-yl)-1,4,5,6-tetrahydropyrimidine (MCD-386) and pharmaceutically acceptable salts and solvates thereof; 3-Methyl-5-(piperidin-3-yl)-1,2,4-oxadiazole, its enantiomers and pharmaceutically acceptable salts and solvates thereof; 3-[(1S,2S)-2-hydroxycyclohexyl]-6-[(6-methylpyridin-3-yl)methyl]benzo[h] quinazolin-4(3H)-one (MK-7622) and pharmaceutically acceptable salts and solvates thereof, and
   (c) an AChEI selected from the group consisting of donepezil and pharmaceutically acceptable salts thereof, rivastigmine and pharmaceutically acceptable salts thereof, and galantamine and pharmaceutically acceptable salts thereof,
   in admixture with a pharmaceutical carrier; and
(b) a pharmaceutical composition in dosage unit form comprising or consisting essentially of a naAEA selected from the group consisting of alosetron and pharmaceutically acceptable salts and solvates thereof, especially its hydrochloride; azasetron and pharmaceutically acceptable salts and solvates thereof, especially its hydrochloride; ondansetron and pharmaceutically acceptable salts and solvates thereof, especially its monohydrochloride dihydrate; granisetron and pharmaceutically acceptable salts and solvates thereof, especially its hydrochloride; dolasetron and pharmaceutically acceptable salts and solvates thereof, especially its monomethanesulfonate monohydrate, ramosetron and pharmaceutically acceptable salts and solvates thereof, especially its hydrochloride; tropisetron and pharmaceutically acceptable salts and solvates thereof, especially its hydrochloride; palonosetron and pharmaceutically acceptable salts and solvates thereof, especially its hydrochloride; domperidone and pharmaceutically acceptable salts and solvates thereof, especially its maleate; metoclopramide and pharmaceutically acceptable salts and solvates thereof, especially its hydrochloride monohydrate; bromopride and pharmaceutically acceptable salts and solvates thereof, especially its monohydrochloride or the dihydrochloride monohydrate; alizapride and pharmaceutically acceptable salts and solvates thereof, especially its hydrochloride; clebopride and pharmaceutically acceptable salts and solvates thereof, especially its malate and the hydrochloride monohydrate; meclizine (meclozine) and pharmaceutically acceptable salts and solvates thereof, especially its hydrochloride monohydrate; promethazine and pharmaceutically acceptable salts and solvates thereof, especially its hydrochloride; chlorpromazine and pharmaceutically acceptable salts and solvates thereof, especially its hydrochloride; prochlorperazine and pharmaceutically acceptable salts and solvates thereof, especially its dimaleate, its dimesylate and its the 1,2-ethanedisulfonate (1:1) (edisilate); hydroxyzine and pharmaceutically acceptable salts and solvates thereof such as the dihydrochloride or the 1,1'-methylene bis(2-hydroxy-3-naphthalenecarboxylic acid (pamoate) dronabinol; nabilone; aprepitant; netupitant; rolapitant; and casopitant; in admixture with a pharmaceutical carrier.

Another advantageous kit according to this third embodiment provides a kit comprising:
(a/c) a novel fixed-dose combination that is a pharmaceutical composition comprising or consisting essentially of
   (a) a MCRA selected from the group consisting of cevimeline hydrochloride hemihydrate, in an amount of from 34.5 mg to 180 mg, xanomeline, as free base, as oxalate or as L-tartrate, in an amount of from 90 mg to 450 mg, milameline hydrochloride, in an amount of from 2.4 mg to 12 mg and MK 7622, as free base, as hydrochloride, as fumarate or as methanesulfonate, in an amount selected from the group consisting of from more than 5 mg to 15 mg, from more than 15 mg to 45 mg, from more than 45 mg to 270 mg, from more than 15 mg to 225 mg, from more than 45 mg to 225 mg, and from 54 mg to 180 mg, and
   (c) an AChEI selected from the group consisting of donepezil hydrochloride, in an amount of from 5 mg to 40 mg; rivastigmine hydrogen tartrate, in an amount selected from the group consisting of from 1 mg to 36 mg; and galantamine hydrobromide, in an amount of from 4 mg to 48 mg,
   in admixture with a pharmaceutical carrier; and
(b) a pharmaceutical composition in dosage unit form comprising or consisting essentially of a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg, in admixture with a pharmaceutical carrier.

In this kit, preferably, the above Component (a/c) is a pharmaceutical composition in dosage unit form wherein said naAEA and said AChEI are mixed together and with a pharmaceutical carrier in an oral IR- or ER-formulation, according to "The Formulations" section below.

According to another particular aspect of this third embodiment, the above Component (b/c) is a pharmaceutical composition in dosage unit form wherein said naAEA is granisetron and said AChEI is rivastigmine in patch releasing from 0.5mg/24h to 3mg/24h granisetron and from 4.6mg/24h to 52mg/24h rivastigmine, according to "The Formulations" section below.

A further advantageous kit according to this third embodiment provides a kit comprising:
(a/c) a fixed-dose combination that is a pharmaceutical composition comprising or consisting essentially of
   (a) a MCRA selected from the group consisting of xanomeline and pharmaceutically acceptable salts and solvates thereof, and
   (c) an AChEI selected from the group consisting of rivastigmine and pharmaceutically acceptable salts and solvates thereof,
   in admixture with a pharmaceutical carrier or vehicle in a TTS; and
(b) a pharmaceutical composition in dosage unit form comprising or consisting essentially of a naAEA selected from the group consisting of alosetron and pharmaceutically acceptable salts and solvates thereof, especially its hydrochloride; azasetron and pharmaceutically acceptable salts and solvates thereof, especially its hydrochloride; ondansetron and pharmaceutically acceptable salts and solvates thereof, especially its monohydrochloride dihydrate; granisetron and pharmaceutically acceptable salts and solvates thereof, especially its hydrochloride; dolasetron and pharmaceutically acceptable salts and solvates thereof, especially its monomethanesulfonate monohydrate, ramosetron and pharmaceutically acceptable salts and solvates thereof, especially its hydrochloride; tropisetron and pharmaceutically acceptable salts and solvates thereof, especially its hydrochloride; palonosetron and pharmaceutically acceptable salts and solvates thereof, especially its hydrochloride; domperidone and pharmaceutically acceptable salts and solvates thereof, especially its maleate; metoclopramide and pharmaceutically acceptable salts and solvates thereof, especially its hydrochloride monohydrate; bromopride and pharmaceutically acceptable salts and solvates thereof, especially its monohydrochloride or the dihydrochloride monohydrate; alizapride and pharmaceutically acceptable salts and solvates thereof, especially its hydrochloride; clebopride and pharmaceutically acceptable salts and solvates thereof, especially its malate and the hydrochloride monohydrate; meclizine (meclozine) and pharmaceutically acceptable salts and solvates thereof, especially its hydrochloride monohydrate; promethazine and pharmaceutically acceptable salts and solvates thereof, especially its hydrochloride; chlorpromazine and pharmaceutically acceptable salts and solvates thereof, especially its hydrochloride; prochlorperazine and pharmaceutically acceptable salts and solvates thereof, especially its dimaleate, its dimesylate and its the 1,2-ethanedisulfonate (1:1) (edisilate); hydroxyzine and pharmaceutically acceptable salts and solvates thereof such as the dihydrochloride or the 4-[(3-Carboxy-2-hydroxynaphthalen-1-yl)methyl]-3-hydroxynaphthalene-2-carboxylate(pamoate); dronabinol; nabilone; aprepitant; netupitant; rolapitant; and casopitant; in admixture with a pharmaceutical carrier.

A particularly advantageous kit according to this third embodiment provides a kit comprising:
(a/c) a fixed-dose combination that is a pharmaceutical composition comprising or consisting essentially of
   (a) a MCRA selected from the group consisting of xanomeline and pharmaceutically acceptable salts and solvates thereof, and
   (c) an AChEI selected from the group consisting of rivastigmine and pharmaceutically acceptable salts and solvates thereof,
   in admixture with a pharmaceutical carrier or vehicle in a rivastigmine and xanomeline TTS, wherein said rivastigmine is released in an amount of from 4.6mg/24h to 52mg/24h; and said xanomeline is released in an amount/24h giving xanomeline plasma concentrations in human from 16.572ng/ml to 78.6ng/ml; and.
(b) a pharmaceutical composition in dosage unit form comprising or essentially consisting of a naAEA selected from the group consisting of alosetron hydrochloride, in an amount (in alosetron) of from 0.25 mg to 6 mg; azasetron hydrochloride, in an amount (in azasetron) of from 5 mg to 60 mg; dolasetron mesylate, in an amount (in dolasetron) of from 25 mg to 600 mg; granisetron hydrochloride, in an amount (in granisetron) of from 0.5 mg to 6 mg; ondansetron hydrochloride dihydrate, in an amount (in ondansetron) of from 2 mg to 64 mg; palonosetron hydrochloride, in an amount (in palonosetron) of from 0.25 mg to 3 mg; ramosetron hydrochloride, in an amount (in ramosetron) of from 0.0125 mg to 0.3 mg, tropisetron hydrochloride, in an amount (in tropisetron) of from 2.5 mg to 30 mg; domperidone and pharmaceutically acceptable salts and solvates thereof, in an amount (in domperidone) of from 5 mg to 60 mg; haloperidol, in an amount of from 0.5 mg to 60 mg; chlorpromazine hydrochloride, in an amount (in chlorpromazine) of from 12.5 mg to 600 mg; prochlorperazine dimaleate, in an amount (in prochlorperazine) of from 2.5 mg to 30 mg; metoclopramide monohydrochloride monohydrate, in an amount (in metoclopramide) of from 5 mg to 60 mg; bromopride monohydrochloride or dihydrochloride monohydrate, in an amount (in bromopride) of from 5 mg to 60 mg; clebopride hydrogen malate or hydrochloride monohydrate, in an amount (in clebopride) of from 0.25 mg to 3 mg; levosulpiride, in an amount of from 12.5 mg to 600 mg; alizapride hydrochloride, in an amount (in alizapride) of from 25 mg to 300 mg; trimethobenzamide monohydrochloride, in an amount (in trimethobenzamide) of from 50 mg to 600 mg; meclizine (also called meclozine) and pharmaceutically acceptable salts and solvates thereof, in an amount (in meclizine) of from 6.25 mg to 300 mg; promethazine hydrochloride, in an amount (in promethazine) of from 12.5 mg to 150 mg; dronabinol in an amount of from 1.25 mg to 60 mg; nabilone, in an amount of from 0.5 mg to 6 mg; aprepitant, in an amount of from 20 mg to 750 mg; netupitant, in an amount of from 150 mg to 1800 mg; rolapitant, in an amount of from 30 mg to 360 mg; and casopitant, in an amount of from 25 mg to 300 mg, in admixture with a pharmaceutical carrier.

According to another particular aspect of this third embodiment, the above Component (a/c), is a pharmaceutical composition in dosage unit form consisting of a patch wherein said MCRA is xanomeline released in an amount/24h providing xanomeline plasma concentrations in human from 16.572 ng/ml to 78.6 ng/ml and said AChEI is rivastigmine released in an amount/24h of from 4.6 mg to 52 mg; and the above Component (b) is a pharmaceutical composition in dosage unit form wherein said naAEA is granisetron in patch releasing from 0.5 mg/24h to 3 mg/24h granisetron.

### The Formulations

The unit form of the present invention may be a tablet, a capsule, a premeasured volume of a liquid solution or suspension for oral administration or a TTS as a gel or patch, including spray patches, for transdermal application. In said unit form the nsPAChA and the MCRA, as free base are as a pharmaceutically acceptable salt or solvate thereof, may be mixed together or separated according to known technologies in admixture with a pharmaceutical carrier in a pharmaceutical composition.

Component (a), Component (b) and optional Component (c) are formulated with conventional pharmaceutical carriers in known formulations for oral use wherein said components are mixed together or separated, for example in two or three tablets introduced in a capsule or in a two-compartment capsule, wherein one of the Components (a) is in a first of the two compartments and Component (b), alone or mixed with Component (c) in the second of the two compartments, or in a multilayer (di-layer or tri-layer) tablet wherein either Component (a) in admixture with a pharmaceutical carrier is in one of the layers and Component (b), alone or mixed with Component (c) and a pharmaceutical carrier is in a second layer in a di-layer tablet or the Components (a), (b) and (c), in admixture with a pharmaceutical carrier, are each distributed, in admixture with a pharmaceutical carrier in the three layers of a tri-layer tablet. The components are all in IR or in ER form or one of the Components is in IR form and the other(s) is/are in ER form, according to known technologies.

The pharmaceutical carriers and vehicles are those commonly used for the preparation of compositions for oral, buccal, including sublingual, and parenteral, in particular transdermal, administration. Appropriate unit forms comprise the oral forms such as tablets, including orodispersible tablets and orosoluble tablets, soft or hard gelatin capsules, powders or granulates in sachets and suitably measured oral solutions or suspensions as well as transdermal therapeutic systems such as patches for transdermal administration.

Component (a), Component (b) and optional Component (c) may also be present in form of one of their complexes with a cyclodextrin, for example α-cyclodextrin, β-cyclodextrin, γ-cyclodextrin, 2-hydroxypropyl-β-cyclodextrin or methyl-β-cyclodextrin.

Component (a), Component (b) and optional Component (c) may also be formulated in the form of microcapsules, optionally with one or more carriers or additives.

For oral administration, Component (a), Component (b) and optional Component (c), together or separately, are formulated by mixing the active ingredient with conventional pharmaceutical acceptable carriers enabling said active ingredients to be formulated in tablets, dragees, orally disintegrating tablets, capsules, liquid solutions or suspensions, syrups and the like.

Carriers for IR tablets may include, but are not limited to, starches, cellulose and derivatives thereof; lubricants such as talc, stearic acid or magnesium stearate; diluents such as talc, powdered cellulose, lactose, starches such as maize or corn starch, mannitol, sorbitol; disaggregating agents such as microcrystalline cellulose or crospovidone; lubricants such as polyethylene glycol or magnesium stearate; ligands such as methylcellulose, sodium carboxymethylcellulose, alginic acid, alginates; sweeteners, such as sucrose, dextrose, mannitol, saccharin; or flavoring agents such as natural or synthetic oils.

Carriers for orally disintegrating tablets may include, but are not limited to, lubricants, aggregating, sweetening, flavoring or disaggregating agents as well as agents improving the buccal mucosa absorption of Component (a), Component (b) and optional Component (c) such as sorbitol, mannitol, lactose and cellulose.

Carriers for liquid, normally aqueous, suspensions or solutions may include, but are not limited to, antioxidants, such as sodium metabisulfite or sodium sulfite, thickening agents, such as microcrystalline cellulose, hydroxypropylcellulose, carboxymethylcellulose or polyvinylpyrrolidone, preservatives such as methyl paraben, ethyl paraben, sodium ethylenediaminotetracetate, sodium benzoate or an alkaline salt of sorbic acid, as well as flavoring and sweetening agents.

The sweeteners contained in the orally disintegrating tablets and the liquid suspensions or solutions may be natural, optional reduced sugars such as sucrose, dextrose, xylitol, mannitol or sorbitol, or synthetic product such as sodium saccharine or aspartame.

The flavoring agents are pharmaceutically acceptable flavors and tastes of synthetic and natural oils, the latter extracted from plants, leaves, flowers, fruits and their combinations, which include, but are not limited to, cinnamon, peppermint, anise and citron leaves, bitter almond, or citrus fruits, in particular orange and/or lemon, linden and grapefruit oils. Also chocolate, vanilla or eucalyptus flavor and essences of fruit, in particular apple, pear, peach, strawberry, cherry, apricot, orange, lemon and/or grapes may be advantageously used.

The composition according to the present invention may be in form of a capsule containing two tablets as described herein above, one of them comprising Component (a), and the other comprising Component (b) and optional Component (c) in admixture with each other and with a pharmaceutical carrier. The unit form may also be a capsule containing two tablets as described herein above, one of them comprising Component (b), and the other comprising Component (a) and optional Component (c) in admixture with each other and with a pharmaceutical carrier. The unit form may also be a capsule containing two tablets as described herein above, one of them comprising Component (c), and the other comprising Component (a) and Component (b) in admixture each other and with a pharmaceutical carrier.

The unit form may also be a capsule containing three tablets as described herein above, one of them comprising Component (a), the second comprising Component (a) and the third comprising Component (c) in admixture with each other and with a pharmaceutical carrier.

The combination may be formulated in tablets in which one or both of the two components (a) and (b) is/are in controlled-release formulation, for example as a dispersion of said component in hydroxypropyl methyl cellulose or in a film-coated microgranule. Advantageously, the MCRA Component (a), in an ER-formulation is in the core and the naAEA Component (b), alone or in combination with the AChEI Component (c), in IR-formulation, are in the outer layer in multi-layer tablets in which, for example, both the core and the outer layers are coated with a film. Analogously, capsules made of two separated compartments, one containing Component (a), in IR- or ER-formulation and the other containing Component (b) and, optionally, Component (c), in ER- or, preferably, in IR-formulation, may be used.

Carriers and vehicles for ER tablets may include, but are not limited to, retardant materials such as acrylic and methacrylic acid polymers and copolymers; cellulose derivatives such as hydroxypropylmethylcellulose, hydroxyethylcellulose, hydroxypropylethylcellulose, hydroxypropylcellulose, methylcellulose, ethylcellulose, or sodium carboxymethylcellulose; gums; waxes; glycerides or aliphatic alcohols or a mixture thereof.

Component (a), Component (b) and Component (c), as the base thereof or as a pharmaceutically acceptable salt thereof, may also be formulated, together or separately in any TTS such as a patch, a gel, a cream, a spray, an ointment, a lotion or a paste. Advantageously, in said TTS, Component (a) or Component (b) or optional Component (c) is present in admixture with the common diluents and permeation enhancers for the TTS administration; or Component (a) and Component (c) are both present in admixture with the common diluents and permeation enhancers for the TTS administration; or the Component (a) and Component (c) are both present in admixture with the common diluents and permeation enhancers, or Component (a), Component (b) and Component (c) are altogether present in admixture with the common diluents and permeation enhancers.

The permeation enhancer may be any compound which allows the improved permeation of drugs through the skin (see for example the review in Pharmaceutical Technology, November 1997, pages 58-66, the disclosure of which is herein incorporated by reference in its entirety). Such substances may include, but are not limited to, be lower (C₁-C₄) alkanols; fatty alcohols such as lauryl alcohol (dodecanol), alone or in combination with a lower alkanol; fatty acids such as linolenic acid or oleic acid; fatty acid esters such as isopropyl palmitate, stearate, linoleate, oleate or myristate; glycerol; glycerol monoesters such as glycerol monostearate, monolinoleate or monooleate; glycerol diesters; glycerol triesters such as triacetin; sucrose monostearate, monolinoleate or monooleate; sorbitan esters; fatty alcohol ethers having from 10 to 20 carbon atoms; glycols, such as diethylene glycol or propylene glycol; or glycols lower alkyl ethers, such as diethylene glycol mono(C₂-C₄)alkyl ether, in particular diethylene glycol monoethyl ether.

These permeation enhancers are present in an amount from 0.01 to 20% by weight of the total weight of the composition, advantageously in an amount of from 0.05 to 10% by weight, preferably from 0.1 to 5% by weight.

Typically, a TTS in form of a patch is manufactured by mixing a predetermined amount of xanomeline Component (a), of granisetron Component (b) or of an association of the two drugs with the aforementioned permeation enhancer in a laminated composite which basically contains at least one reservoir comprising a adhesive which is a pressure-sensitive adhesive suitable for the contact with the skin, a backing layer and a strip to be removed just before the application of the patch on the subject's skin. The xanomeline patch, containing xanomeline alone, may be manufactured for example as illustrated in the above-cited US 5,980,933.

### Use

As set forth herein, Component (a), Component (b) and optional Component (c) may be administered concurrently or sequentially to a patient suffering from a hypocholinergic disorder of the CNS such as Alzheimer type dementia and schizophrenia. In particular, Component (a), Component (b) and optional Component (c) can be administered in a specific dosage regimen as illustrated above - to treat Alzheimer type dementia and schizophrenia, Component (a), Component (b) and optional Component (c) being administered simultaneously or sequentially to one another, in each case by the same or different administration route.

By the combination of Component (a) and Component (b) such as in the same unit form, Component (b) allows for the safe administration of high doses of Component (a) without dangerous adverse effects linked to the peripheral cholinergic action of said Component (a). Accordingly, the therapeutic efficacy of Component (a) to safely improve cognition of patients suffering from a hypocholinergic disorder of the CNS such as Alzheimer type dementia or schizophrenia is enhanced, due to the action of Component (b) annulling vomiting, i.e. the cause of the failure of all the precedent attempts of using a MCRA for the treatment of Alzheimer type dementia and, in general of hypocholinergic disorders of the CNS.

The presence of a naAEA in a combination wherein, besides the MCRA Component (a) and the naAEA Component (b), an AChEI Component (c) is also present, is of great importance

Thus, the present invention in one aspect, provides a combination comprising or consisting essentially of, as Components:
(a) a muscarinic cholinergic receptor agonists (MCRA); and
(b) a non-anticholinergic antiemetic agent; and, optionally,
(c) an acetylcholinesterase inhibitor (AChEI);
for use in the treatment of a hypocholinergic disorder of the CNS.

The MCRAs used as Component (a), their properties and doses are described in the "The MCRAs" section above.

The naAEAs used as Component (b), their properties and doses are described in the "The naAEAs" section above.

The AChEI optionally used as Component (c), their properties and doses are described at the end of "The Combinations" section.

For use, Component (a), Component (b) and optional Component (c), together or separately, are formulated in pharmaceutical compositions prepared as described in the "The Formulations" section above.

Thus, the present invention in one aspect, provides a combination comprising or consisting essentially of, as Components:
(a) a muscarinic cholinergic receptor agonists (MCRA); and
(b) a non-anticholinergic antiemetic agent
for use in the treatment of a hypocholinergic disorder of the CNS.

The MCRAs used as Component (a), their properties and doses are described in the "The MCRAs" section above.

The naAEAs used as Component (b), their properties and doses are described in the "The naAEAs" section above.

For use, Component (a) and Component (b), together or separately, are formulated in pharmaceutical compositions prepared as described in the "The Formulations" section above.

Another aspect of the present invention provides a method for treating hypocholinergic disorders of the central nervous system, comprising, or consisting essentially of, the Components:
(a) a muscarinic cholinergic receptor agonists (MCRA); and
(b) a non-anticholinergic antiemetic agent; and, optionally,
(c) an acetylcholinesterase inhibitor (AChEI).

The method is carried out by administering Component (a), Component (b) and optional Component (c) of said combination concurrently, or sequentially. Component (a), Component (b) and optional Component (c) may be independently administered by oral or parenteral route, in particular by intramuscular or intravenous injection or by transdermal administration by a TTS such as a gel or a patch.

The MCRAs used as Component (a), their properties and doses are described in the "The MCRAs" section above.

The naAEA used as Component (b), their properties and doses are described in the "The naAEAs" section above.

The AChEI optionally used as Component (c), their properties and doses are described at the end of "The Combinations" section.

For administering the combination to said patient, Component (a), Component (b) and optional Component (c), together or separately, are formulated in pharmaceutical compositions prepared as described in the "The Formulations" section above.

In the case of simultaneous administration of the components, Component (a), Component (b) and Component (c), in admixture with a pharmaceutical carrier or vehicle, may be associated in the same pharmaceutical composition, formulated as described in "The Formulations" section above, in a unit dose for oral or parenteral, including transdermal, route, according to known or conventional methods or technologies in the art.

The doses of the above dose-ranges are given in order to present the possibility of manufacturing pharmaceutical compositions and of administering said doses in said pharmaceutical composition. However, the discovery of the beneficial action of the naAEA enables the full efficacy of the MCRA and of the optional AChEI.

### ADDITIONAL EMBODIMENTS

1. A pharmaceutical combination comprising as Components:
   (a) a muscarinic cholinergic receptor agonist (MCRA); and
   (b) a non-anticholinergic antiemetic agent (naAEA).
2. The combination of embodiment 1, wherein said MCRA Component (a) is in a pharmaceutical composition in admixture with a pharmaceutical carrier; and said naAEA Component (b) is in a pharmaceutical composition in admixture with a pharmaceutical carrier.
3. The combination of embodiment 1, wherein said MCRA Component (a) is in a pharmaceutical composition in admixture with a pharmaceutical carrier; and said naAEA Component (b) is selected from the group consisting of ondansetron and pharmaceutically acceptable salts and solvates thereof in a pharmaceutical composition in admixture with a pharmaceutical carrier.
4. The combination of embodiment 3, wherein said MCRA Component (a) is selected from the group consisting of cevimeline and pharmaceutically acceptable salts thereof, milameline and pharmaceutically acceptable salts thereof; xanomeline and pharmaceutically acceptable salts thereof, and MK-7622 and pharmaceutically acceptable salts thereof; and said naAEA Component (b) is ondansetron hydrochloride dihydrate.
5. The combination of embodiment 4, wherein said MCRA Component (a) is selected from the group consisting of cevimeline and pharmaceutically acceptable salts thereof, in an amount (in cevimeline) of from 34.5 mg to 180 mg; milameline and pharmaceutically acceptable salts thereof, in an amount (in milameline) of from 2.4 mg to 12 mg; xanomeline and pharmaceutically acceptable salts thereof, in an amount (in xanomeline) of from 90 mg to 450 mg; and MK-7622 and pharmaceutically acceptable salts thereof, in an amount (in MK-7622) of from 5 mg to 270 mg; in a pharmaceutical composition in admixture with a pharmaceutical carrier; and said naAEA Component (b) is ondansetron hydrochloride dihydrate, in an amount (in ondansetron) of from 4 mg to 64 mg.
6. The combination of embodiment 5, wherein said MCRA Component (a) is cevimeline or a pharmaceutically acceptable salt thereof.
7. A method for treating hypocholinergic disorders of the central nervous system, which comprises administering, to a patient in need of such a treatment, an effective amount of the Component (a) and an effective amount of the Component (b) of the combination according to any one of embodiments 1 to 6.
8. The method of embodiment 7, wherein Component (a) and Component (b) of the combination are administered concurrently or sequentially to a patient suffering from a hypocholinergic disorder of the central nervous system, each Component being administered to said patient by the same or by a different administration route.
9. The method of embodiment 8, wherein said hypocholinergic disorder of the central nervous system is selected from the group consisting of Alzheimer's disease (AD), Alzheimer-type dementia, mild cognitive impairment, Lewy body disease, Parkinson's disease dementia, Frontotemporal lobe dementia (FTD), Frontotemporal lobar degeneration, post-stroke dementia, vascular dementia, traumatic brain injury, Senile dementia, Autism, Down syndrome, anorexia nervosa, Tourette syndrome, tardive dyskinesia, Pick's disease, Huntington's disease, Friedrich's ataxia, chronic neuropathic pain, falls, post-operative delirium, schizophrenia, Cognitive Impairment associated with Multiple Sclerosis, and other disorders of the nervous system involving a deficit in acetyl-choline neurotransmission.
10. The combination according to any one of embodiments 1 to 6, for use in the treatment of hypocholinergic disorders of the CNS.
11. The combination of embodiment 10, wherein said hypocholinergic disorder of the central nervous system is selected from the group consisting of Alzheimer's disease (AD), Alzheimer-type dementia, mild cognitive impairment, Lewy body disease, Parkinson's disease dementia, Frontotemporal lobe dementia (FTD), Frontotemporal lobar degeneration, post-stroke dementia, vascular dementia, traumatic brain injury, Senile dementia, Autism, Down syndrome, anorexia nervosa, Tourette syndrome, tardive dyskinesia, Pick's disease, Huntington's disease, Friedrich's ataxia, chronic neuropathic pain, falls, post-operative delirium, schizophrenia, Cognitive Impairment associated with Multiple Sclerosis, and other disorders of the nervous system involving a deficit in acetyl-choline neurotransmission.
12. The combination of embodiment 1, further comprising, as a Component:
   (c) an acetyl choline esterase inhibitor (AChEI).

## Claims

1. A pharmaceutical combination comprising as Components:
(a) a muscarinic cholinergic receptor agonist (MCRA); and
(b) a non-anticholinergic antiemetic agent (naAEA).

2. The combination of claim 1, wherein said MCRA Component (a) is in a pharmaceutical composition in admixture with a pharmaceutical carrier; and said naAEA Component (b) is in a pharmaceutical composition in admixture with a pharmaceutical carrier.

3. The combination of claim 1, wherein said MCRA Component (a) is in a pharmaceutical composition in admixture with a pharmaceutical carrier; and said naAEA Component (b) is selected from the group consisting of ondansetron and pharmaceutically acceptable salts and solvates thereof in a pharmaceutical composition in admixture with a pharmaceutical carrier.

4. The combination of claim 3, wherein said MCRA Component (a) is selected from the group consisting of cevimeline and pharmaceutically acceptable salts thereof, milameline and pharmaceutically acceptable salts thereof; xanomeline and pharmaceutically acceptable salts thereof, and MK-7622 and pharmaceutically acceptable salts thereof; and said naAEA Component (b) is ondansetron hydrochloride dihydrate.

5. The combination of claim 4, wherein said MCRA Component (a) is selected from the group consisting of cevimeline and pharmaceutically acceptable salts thereof, in an amount (in cevimeline) of from 34.5 mg to 180 mg; milameline and pharmaceutically acceptable salts thereof, in an amount (in milameline) of from 2.4 mg to 12 mg; xanomeline and pharmaceutically acceptable salts thereof, in an amount (in xanomeline) of from 90 mg to 450 mg; and MK-7622 and pharmaceutically acceptable salts thereof, in an amount (in MK-7622) of from 5 mg to 270 mg; in a pharmaceutical composition in admixture with a pharmaceutical carrier; and said naAEA Component (b) is ondansetron hydrochloride dihydrate, in an amount (in ondansetron) of from 4 mg to 64 mg.

6. The combination of claim 5, wherein said MCRA Component (a) is cevimeline or a pharmaceutically acceptable salt thereof.

7. The combination of claim 1, further comprising, as a Component:
(c) an acetyl choline esterase inhibitor (AChEI).

8. A combination according to any one of claims 1-7, for treating hypocholinergic disorders of the central nervous system.

9. The combination of claim 8, wherein Component (a) and Component (b) of the combination are administered concurrently or sequentially to a patient suffering from a hypocholinergic disorder of the central nervous system, each Component being administered to said patient by the same or by a different administration route.

10. The combination of claim 9, wherein said hypocholinergic disorder of the central nervous system is selected from the group consisting of Alzheimer's disease (AD), Alzheimer-type dementia, mild cognitive impairment, Lewy body disease, Parkinson's disease dementia, Frontotemporal lobe dementia (FTD), Frontotemporal lobar degeneration, post-stroke dementia, vascular dementia, traumatic brain injury, Senile dementia, Autism, Down syndrome, anorexia nervosa, Tourette syndrome, tardive dyskinesia, Pick's disease, Huntington's disease, Friedrich's ataxia, chronic neuropathic pain, falls, post-operative delirium, schizophrenia, Cognitive Impairment associated with Multiple Sclerosis, and other disorders of the nervous system involving a deficit in acetyl-choline neurotransmission.

11. The combination according to any one of claims 1 to 10, for use in the treatment of hypocholinergic disorders of the CNS.

12. The combination for use according to claim 11, wherein said hypocholinergic disorder of the central nervous system is selected from the group consisting of Alzheimer's disease (AD), Alzheimer-type dementia, mild cognitive impairment, Lewy body disease, Parkinson's disease dementia, Frontotemporal lobe dementia (FTD), Frontotemporal lobar degeneration, post-stroke dementia, vascular dementia, traumatic brain injury, Senile dementia, Autism, Down syndrome, anorexia nervosa, Tourette syndrome, tardive dyskinesia, Pick's disease, Huntington's disease, Friedrich's ataxia, chronic neuropathic pain, falls, post-operative delirium, schizophrenia, Cognitive Impairment associated with Multiple Sclerosis, and other disorders of the nervous system involving a deficit in acetyl-choline neurotransmission.
